# EUROPÄISCHE PATENTSCHRIFT

(11) **EP 1 715 883 B1**
(45) Veröffentlichungstag und Bekanntmachung des Hinweises auf die Patenterteilung: **22.04.2009**
(21) Anmeldenummer: 04803585.1
(22) Anmeldetag: 07.12.2004
(51) Int. Cl.: A61K 38/15, A61K 31/395, C07K 11/02, C07D 273/00, A61P 33/10

(54) **18-GLIEDRIGE NITROBENZYL- UND AMINOBENZYL-SUBSTITUIERTE CYCLOHEXADEPSIPEPTIDE ZUR BEKÄMPFUNG VON ENDOPARASITEN UND EIN VERFAHREN ZU IHRER HERSTELLUNG**
18-MEMBERED NITROBENZYL-SUBSTITUTED AND AMINOBENZYL-SUBSTITUTED CYCLOHEXADEPSIPEPTIDES FOR CONTROLLING ENDOPARASITES, AND METHOD FOR THE PRODUCTION THEREOF
CYCLOHEXADEPSIPEPTIDES A 18 CHAINONS ET A SUBSTITUTION NITROBENZYLE ET AMINOBENZYLE POUR LUTTER CONTRE DES ENDOPARASITES ET PROCEDE POUR LEUR PRODUCTION

(30) Priorität: 19.12.2003 DE 10359798
(43) Veröffentlichungstag der Anmeldung: 02.11.2006
(73) Patentinhaber: Bayer Animal Health GmbH, 51368 Leverkusen (DE)
(72) Erfinder: JESCHKE, Peter, 51467 Bergisch Gladbach (DE); HARDER, Achim, 51109 Köln (DE)
(86) Internationale Anmeldenummer: PCT/EP2004/013896
(87) Internationale Veröffentlichungsnummer: WO 2005/063277

(56) Entgegenhaltungen:
- EP-A- 0 662 326
- EP-A- 0 664 297
- WO-A-95/27498
- WO-A-96/38165

## Beschreibung

Die vorliegende Erfindung betrifft cyclische Depsipeptide, insbesondere 18-gliedrige Cyclohexadepsipeptide, ein Verfahren zu ihrer Herstellung und ihre Verwendung zur Bekämpfung von Endoparasiten.

Verschiedene Cyclodepsipeptide mit 18 Ringatomen als Mittel zur Bekämpfung von Endoparasiten sind bereits bekannt geworden (vgl. z. B. DE 4 317 458 A1, EP 669 343 A1, EP 658 551 A1).

EP-A-0662 326, EP-A-0664 297, WO 95/27498 und WO 96/38165 offenbaren weitere cyclische Depsipeptide zur Bekämpfung von Endoparasiten.

Die Wirksamkeit dieser vorbekannten Verbindungen ist jedoch bei niedrigen Aufwandmengen und Konzentrationen nicht völlig zufriedenstellend.

Gegenstand der vorliegenden Erfindung sind neue cyclische Depsipeptide und Verfahren zur Darstellung der cyclischen Depsipeptide, bestehend aus Aminosäuren und Hydroxycarbonsäuren als Ringbausteine und 18 Ringatomen.

Ein weiterer Gegenstand ist auch die Verwendung von cyclischen Depsipeptiden, bestehend aus Aminosäuren und Hydroxycarbonsäuren als Ringbausteine und 18 Ringatomen, zur Herstellung von Mitteln zur Bekämpfung von Endoparasiten.

Die vorliegende Erfindung betrifft insbesondere:
1. Cyclische Depsipeptide der allgemeinen Formel (I) und deren Salze, 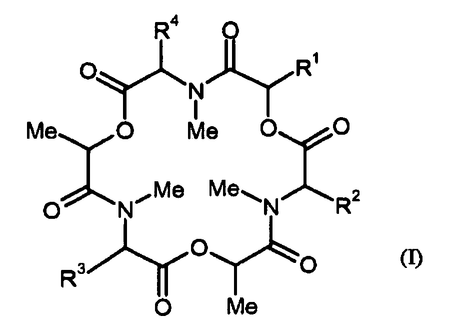 in welcher
   - R¹: für Nitrobenzyl oder R'R" N-Benzyl steht,
   worin
   - R' und R": unabhängig voneinander jeweils Wasserstoff, gegebenenfalls substituiertes C₁-C₄-Alkyl, Formyl, C₁-C₄-Alkoxy-C₁-C₄-alkyl, C₁-C₄-Alkoxycarbonyl, Hydroxy-C₁-C₂-alkyl-sulfonyl-C₁-C₂-alkyl bedeuten
   oder
   - R' und R": gemeinsam mit dem Stickstoffatom, an das sie gebunden sind, einen gegebenenfalls substituierten mono- oder polycyclischen, gegebenenfalls überbrückten und/oder spirocyclischen, gesättigten oder ungesättigten Heterocyclus bilden, der ein bis drei weitere Heteroatome aus der Reihe Stickstoff, Sauerstoff und Schwefel enthält, oder 'R' und R" gemeinsam C₃-C₅-Alkylenmonocarbonyl oder einen gegebenenfalls substituierten Diacylrest einer C₄-C₆-Dicarbonsäure bilden, und
   - R², R³ und R⁴: unabhängig voneinander für C₁-C₄-Alkyl stehen,
   sowie deren optische Isomere und Racemate.
2. Die neuen cyclischen Depsipeptide der allgemeinen Formel (I) und deren Salze 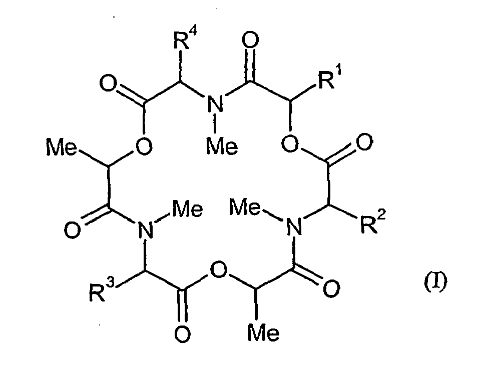 in welcher
   R¹, R², R³ und R⁴ die unter Punkt 1 angegebene Bedeutung besitzen,
   werden hergestellt, indem man
   a) in einem ersten Schritt die cyclischen Depsipeptide der allgemeinen Formel (II] und deren Salze, 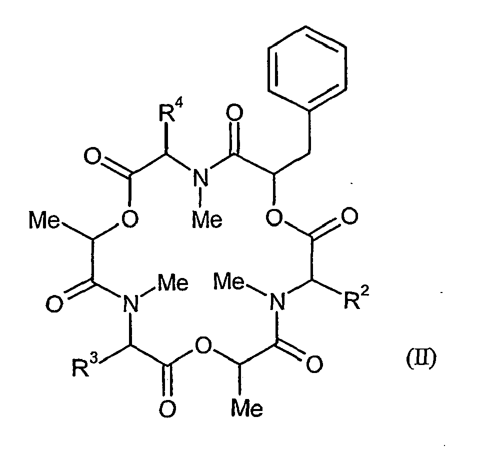 in welcher
      R², R³ und R⁴ die unter Punkt 1 angegebene Bedeutung besitzen,
      in Gegenwart eines Nitrierungsmittels und gegebenenfalls in Gegenwart eines Verdünnungsmittels nitriert, und
   b) gegebenenfalls in einem zweiten Schritt in den so erhaltenen cyclischen Depsipeptiden der allgemeinen Formel (III] oder deren Salzen, 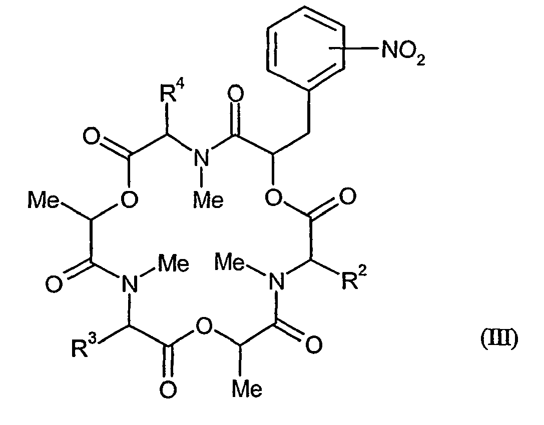 in welcher
      R², R³ und R⁴ die unter Punkt 1 angegebene Bedeutung besitzen,
      in Gegenwart eines Reduktionsmittels und gegebenenfalls in Gegenwart eines Verdünnungsmittels die Nitrogruppe reduziert, und
   c) gegebenenfalls in einem dritten Schritt die cyclischen Depsipeptide der allgemeinen Formel (IV) und deren Salze, 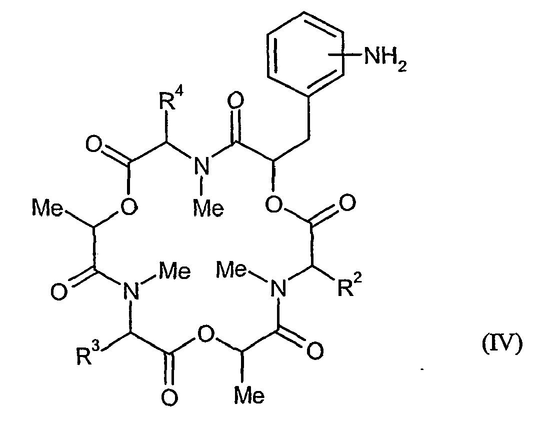
   in welcher
   R², R³ und R⁴ die unter Punkt 1 angegebene Bedeutung besitzen,
   zur Einführung der Reste R' und R" in Gegenwart eines geeigneten Aldehyds und eines Reduktionsmittels und gegebenenfalls in Gegenwart eines Verdünnungsmittels aminoalkyliert, oder
   in Gegenwart eines geeigneten Alkylierungsmittels und eines basischen Reaktionshilfsmittels und gegebenenfalls in Gegenwart eines Verdünnungsmittels N-alkyliert, oder
   in Gegenwart eines geeigneten Acylierungsmittels und eines basischen Reaktionshilfsmittels und gegebenenfalls in Gegenwart eines Verdünnungsmittels N-acyliert.

Die Verbindungen der allgemeinen Formel (I) können in Abhängigkeit von der Art der Substituenten als geometrische und/oder optische Isomerengemische unterschiedlicher Zusammensetzung auftreten. Die Erfindung betrifft sowohl die reinen Isomeren als auch Isomerengemische.

Bevorzugt sind cyclische Depsipeptide, bestehend aus Aminosäuren und Hydroxycarbonsäuren als Ringbausteine und 18 Ringatomen, der allgemeinen Formel (I) und deren Salze 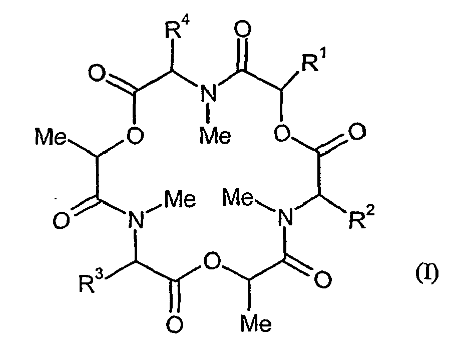 in welcher
- R¹: für Nitrobenzyl oder R'R"N-Benzyl stehen,
worin
- R' und R": unabhängig voneinander jeweils für Wasserstoff, C₁-C₃-Alkyl, insbesondere Methyl, Ethyl, C₁-C₃-Alkoxy-C₁-C₃-Alkyl, insbesondere Methoxyethyl, 2-Hydroxyethylsulfonyl-C₁-C₂-alkyl insbesondere 2-Hydroxyethyl-sulfonyl-ethyl oder
- R' und R": gemeinsam mit dem Stickstoffatom, an das sie gebunden sind, N-Pyrrolidino, N-Piperidino, N-Piperazino, N-Morpholino, N-2,6-Dimethylmorpholino, N-Thiomorpholino, N-Pyrazolo, N-Imidazolo, 2-Oxo-pyrrolidin-1-yl, 2-Oxo-piperidin-1-yl, 2-Oxo-azepan-1-yl-methyl, Succinimino, Maleinimino oder Glutarimino bedeuten,
- R², R³ und R⁴: unabhängig voneinander für C₁-C₄-Alkyl stehen,
sowie deren optische Isomere und Racemate.

Besonders bevorzugt sind cyclische Depsipeptide, bestehend aus Aminosäuren und Hydroxycarbonsäuren als Ringbausteine und 18 Ringatomen, der allgemeinen Formel (I) und deren Salze 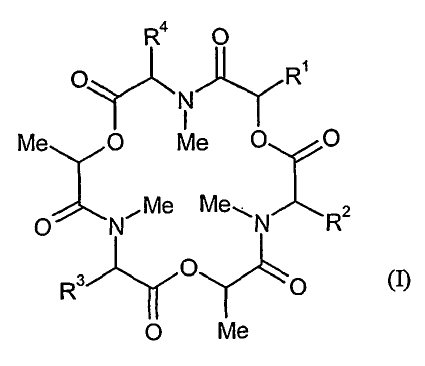 in welcher
- R¹: für 4-Nitrobenzyl, 4-Aminobenzyl, 4-Morpholinobenzyl, 4-Hydroxyethyl-sulfonyl-ethylaminobenzyl
- R² und R⁴: unabhängig voneinander für C₁-C₄-Alkyl, insbesondere Methyl, Isopropyl, Isobutyl oder sek-Butyl stehen,
- R³: für Methyl oder Ethyl steht,
sowie deren optische Isomere und Racemate.

Ganz besonders bevorzugt sind cyclische Depsipeptide, bestehend aus Aminosäuren und Hydroxycarbonsäuren als Ringbausteine und 18 Ringatomen, der allgemeinen Formel ( I ) und deren Salze 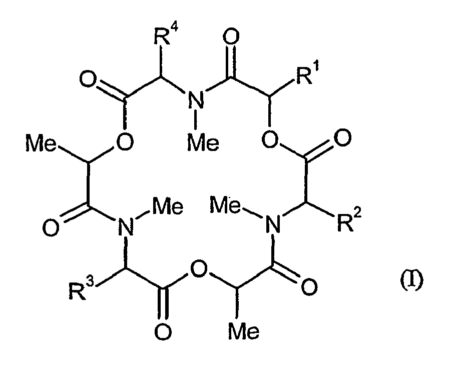 in welcher
- R¹: für 4-Nitrobenzyl, 4-Aminobenzyl, 4-Morpholinobenzyl, 4-Hydroxyethylsulfonyl-ethylaminobenzyl,
- R² und R⁴: für sek-Butyl stehen,
- R³: für Methyl steht,
sowie deren optische Isomere und Racemate.

Die erfindungsgemäßen cyclischen Depsipeptide und deren Säureadditionssalze und Metallsalzkomplexe besitzen gute endoparasitizide, insbesondere anthelmintische Wirkung und können bevorzugt im Bereich der Veterinärmedizin eingesetzt werden.

Die erfindungsgemäßen cyclischen Depsipeptide der allgemeinen Formel (I) und deren Salze enthalten ein oder mehrere Chiralitätszentren und können somit als reine Stereoisomere oder in Form von verschiedenen Enantiomeren- und Diastereomerengemische vorliegen, die erforderlichenfalls in an sich bekannter Weise getrennt werden können oder auch durch stereoselektive Reaktionen in Kombination mit dem Einsatz von stereochemisch reinen Ausgangsstoffen hergestellt werden.

Vorzugsweise werden jedoch die optisch aktiven, stereoisomeren Formen der Verbindungen der allgemeinen Formel (I) und deren Salze erfindungsgemäß verwendet. Besonders bevorzugt sind die cyclischen Depsipeptide, die sich aus (S)-konfigurierten Aminosäuren (L-Form) und (R)-konfigurierten Hydroxycarbonsäuren (D-Form) als Ringbausteine zusammensetzen.

Die Erfindung betrifft daher sowohl die reinen Enantiomeren und Diastereomeren, als auch deren Gemische zur Bekämpfung von Endoparasiten, besonders auf dem Gebiet der Medizin und Veterinärmedizin.

Als geeignete Salze der Depsipeptide der allgemeinen Formel (I) können übliche nicht toxische Salze, d. h. Salze mit entsprechenden Basen und Salze mit zugesetzten Säuren genannt werden. Vorzugsweise sind Salze mit anorganischen Basen, wie Alkalimetallsalze, beispielsweise Natrium-, Kalium- oder Cäsiumsalze, Erdalkalimetallsalze, beispielsweise Calzium- oder Magnesiumsalze, Ammoniumsalze, Salze mit organischen Basen sowie mit anorganischen Aminen, beispielsweise Triethylammonium-, Dicyclohexylammonium-, N,N'-Dibenzylethylendiammonium-, Pyridinium-, Picolinium- oder Ethanolammoniumsalze, Salze mit anorganischen Säuren, beispielsweise Hydrochloride, Hydrobromide, Dihydrosulfate, Trihydrosulfate, oder Phosphate, Salze mit organischen Carbonsäuren oder organischen Sulfosäure, beispielsweise Formiate, Acetate, Trifluoracetate, Maleate, Tartrate, Methansulfonate, Benzolsulfonate oder para-Toluolsulfonate, Salze mit basischen Aminosäuren, beispielsweise Arginate, Aspartate oder Glutamate und Ähnliches zu nennen.

Weiterhin gehören zu den Salzen der Depsipeptide auch Metallsalzkomplexe, beispielsweise Alkalimetallsalze wie Natrium-, Kalium- oder Caesiumsalze oder Erdalkalimetallsalze wie beispielsweise Calzium- oder Magnesiumsalze.

Die Depsipeptide oder ihre Salze können als Feststoffe auch in Form von Solvaten, insbesondere Hydraten vorliegen. Diese werden erfindungsgemäß ebenfalls umfasst.

Im einzelnen seien die nachfolgenden Cyclodepsipeptide mit 18 Ringatomen genannt:
Cyclo(-N-methyl-L-alanyl-D-lactyl-N-methyl-L-isoleucyl-D-2-nitro-phenyllactyl-N-methyl-L-isoleucyl-D-lactyl-),
Cyclo(-N-methyl-L-alanyl-D-lactyl-N-methyl-L-isoleucyl-D-3-nitro-phenyllactyl-N-methyl-L-isoleucyl-D-lactyl-),
Cyclo(-N-methyl-L-alanyl-D-lactyl-N-methyl-L-isoleucyl-D-4-nitro-phenyllactyl-N-methyl-L-isoleucyl-D-lactyl-),
Cyclo(-N-methyl-L-alanyl-D-lactyl-N-methyl-L-isoleucyl-D-2-amino-phenyllactyl-N-methyl-L-isoleucyl-D-lactyl-),
Cyclo(-N-methyl-L-alanyl-D-lactyl-N-methyl-L-isoleucyl-D-3-amino-phenyllactyl-N-methyl-L-isoleucyl-D-lactyl-),
Cyclo(-N-methyl-L-alanyl-D-lactyl-N-methyl-L-isoleucyl-D-4-amino-phenyllactyl-N-methyl-L-isoleucyl-D-lactyl-),
Cyclo(-N-methyl-L-alanyl-D-lactyl-N-methyl-L-isoleucyl-D-3-morpholino-phenyllactyl-N-methyl-L-isoleucyl-D-lactyl-),
Cyclo(-N-methyl-L-alanyl-D-lactyl-N-methyl-L-isoleucyl-D-4-morpholino-phenyllactyl-N-methyl-L-isoleucyl-D-lactyl-),
Cyclo(-N-methyl-L-alanyl-D-lactyl-N-methyl-L-isoleucyl-D-3-(2-hydroxyethylsulfonylethylamino-phenyl)lactyl-N-methyl-L-isoleucyl-D-lactyl-),
Cyclo(-N-methyl-L-alanyl-D-lactyl-N-methyl-L-isoleucyl-D-4-(2-hydroxyethylsulfonylethylamino-phenyl)lactyl-N-methyl-L-isoleucyl-D-lactyl-),
Cyclo(-N-methyl-L-alanyl-D-lactyl-N-methyl-L-valinyl-D-2-nitro-phenyllactyl-N-methyl-L-isoleucyl-D-lactyl-),
Cyclo(-N-methyl-L-alanyl-D-lactyl-N-methyl-L-valinyl-D-3-nitro-phenyllactyl-N-methyl-L-isoleucyl-D-lactyl-),
Cyclo(-N-methyl-L-alanyl-D-lactyl-N-methyl-L-valinyl-D-4-nitro-phenyllactyl-N-methyl-L-isoleucyl-D-lactyl-),
Cyclo(-N-methyl-L-alanyl-D-lactyl-N-methyl-L-valinyl-D-2-amino-phenyllactyl-N-methyl-L-isoleucyl-D-lactyl-),
Cyclo(-N-methyl-L-alanyl-D-lactyl-N-methyl-L-valinyl-D-3-amino-phenyllactyl-N-methyl-L-isoleucyl-D-lactyl-),
Cyclo(-N-methyl-L-alanyl-D-lactyl-N-methyl-L-valinyl-D-4-amino-phenyllactyl-N-methyl-L-isoleucyl-D-lactyl-),
Cyclo(-N-methyl-L-alanyl-D-lactyl-N-methyl-L-valinyl-D-3-morpholino-phenyllactyl-N-methyl-L-isoleucyl-D-lactyl-),
Cyclo(-N-methyl-L-alanyl-D-lactyl-N-methyl-L-valinyl-D-4-morpholino-phenyllactyl-N-methyl-L-isoleucyl-D-lactyl-),
Cyclo(-N-methyl-L-alanyl-D-lactyl-N-methyl-L-valinyl-D-3-(2-hydroxyethylsulfonyl-ethylaminophenyl)lactyl-N-methyl-L-isoleucyl-D-lactyl-),
Cyclo(-N-methyl-L-alanyl-D-lactyl-N-methyl-L-valinyl-D-4-(2-hydroxyethylsulfonyl-ethylaminophenyl)lactyl-N-methyl-L-isoleucyl-D-lactyl-),
Cyclo(-N-methyl-L-2-amino-butyl-D-lactyl-N-methyl-L-isoleucyl-D-2-nitro-phenyllactyl-N-methyl-L-isoleucyl-D-lactyl-),
Cyclo(-N-methyl-L-2-amino-butyl-D-lactyl-N-methyl-L-isoleucyl-D-3-nitro-phenyllactyl-N-methyl-L-isoleucyl-D-lactyl-),
Cyclo(-N-methyl-L-2-amino-butyl-D-lactyl-N-methyl-L-isoleucyl-D-4-nitro-phenyllactyl-N-methyl-L-isoleucyl-D-lactyl-),
Cyclo(-N-methyl-L-2-amino-butyl-D-lactyl-N-methyl-L-isoleucyl-D-2-amino-phenyllactyl-N-methyl-L-isoleucyl-D-lactyl-),
Cyclo(-N-methyl-L-2-amino-butyl-D-lactyl-N-methyl-L-isoleucyl-D-3-amino-phenyllactyl-N-methyl-L-isoleucyl-D-lactyl-),
Cyclo(-N-methyl-L-2-amino-butyl-D-lactyl-N-methyl-L-isoleucyl-D-4-amino-phenyllactyl-N-methyl-L-isoleucyl-D-lactyl-),
Cyclo(-N-methyl-L-2-amino-butyl-D-lactyl-N-methyl-L-isoleucyl-D-3-morpholino-phenyllactyl-N-methyl-L-isoleucyl-D-lactyl-),
Cyclo(-N-methyl-L-2-amino-butyl-D-lactyl-N-methyl-L-isoleucyl-D-4-morpholino-phenyllactyl-N-methyl-L-isoleucyl-D-lactyl-),
Cyclo(-N-methyl-L-2-amino-butyl-D-lactyl-N-methyl-L-isoleucyl-D-3-(2-hydroxyethylsulfonylethylamino-phenyl)lactyl-N-methyl-L-isoleucyl-D-lactyl-),
Cyclo(-N-methyl-L-2-amino-butyl-D-lactyl-N-methyl-L-isoleucyl-D-4-(2-hydroxyethylsulfonylethylamino-phenyl)lactyl-N-methyl-L-isoleucyl-D-lactyl-),
Cyclo(-N-methyl-L-2-amino-butyl-D-lactyl-N-methyl-L-valinyl-D-2-nitro-phenyllactyl-N-methyl-L-isoleucyl-D-lactyl-),
Cyclo(-N-methyl-L-2-amino-butyl-D-lactyl-N-methyl-L-valinyl-D-3-nitro-phenyllactyl-N-methyl-L-isoleucyl-D-lactyl-),
Cyclo(-N-methyl-L-2-amino-butyl-D-lactyl-N-methyl-L-valinyl-D-4-nitro-phenyllactyl-N-methyl-L-isoleucyl-D-lactyl-),
Cyclo(-N-methyl-L-2-amino-butyl-D-lactyl-N-methyl-L-valinyl-D-2-amino-phenyllactyl-N-methyl-L-isoleucyl-D-lactyl-),
Cyclo(-N-methyl-L-2-amino-butyl-D-lactyl-N-methyl-L-valinyl-D-3-amino-phenyllactyl-N-methyl-L-isoleucyl-D-lactyl-),
Cyclo(-N-methyl-L-2-amino-butyl-D-lactyl-N-methyl-L-valinyl-D-4-amino-phenyllactyl-N-methyl-L-isoleucyl-D-lactyl-),
Cyclo(-N-methyl-L-2-amino-butyl-D-lactyl-N-methyl-L-valinyl-D-3-morpholino-phenyllactyl-N-methyl-L-isoleucyl-D-lactyl-),
Cyclo(-N-methyl-L-2-amino-butyl-D-lactyl-N-methyl-L-valinyl-D-4-morpholino-phenyllactyl-N-methyl-L-isoleucyl-D-lactyl-),
Cyclo(-N-methyl-L-2-amino-butyl-D-lactyl-N-methyl-L-valinyl-D-3-(2-hydroxyethylsulfonylethylamino-phenyl)lactyl-N-methyl-L-isoleucyl-D-lactyl-),
Cyclo(-N-methyl-L-2-amino-butyl-D-lactyl-N-methyl-L-valinyl-D-4-(2-hydroxyethylsulfonylethylamino-phenyl)lactyl-N-methyl-L-isoleucyl-D-lactyl-),
Cyclo(-N-methyl-L-alanyl-D-lactyl-N-methyl-L-isoleucyl-D-2-nitro-phenyllactyl-N-methyl-L-valinyl-D-lactyl-),
Cyclo(-N-methyl-L-alanyl-D-lactyl-N-methyl-L-isoleucyl-D-3-nitro-phenyllactyl-N-methyl-L-valinyl-D-lactyl-),
Cyclo(-N-methyl-L-alanyl-D-lactyl-N-methyl-L-isoleucyl-D-4-nitro-phenyllactyl-N-methyl-L-valinyl-D-lactyl-),
Cyclo(-N-methyl-L-alanyl-D-lactyl-N-methyl-L-isoleucyl-D-2-amino-phenyllactyl-N-methyl-L-valinyl-D-lactyl-),
Cyclo(-N-methyl-L-alanyl-D-lactyl-N-methyl-L-isoleucyl-D-3-amino-phenyllactyl-N-methyl-L-valinyl-D-lactyl-),
Cyclo(-N-methyl-L-alanyl-D-lactyl-N-methyl-L-isoleucyl-D-4-amino-phenyllactyl-N-methyl-L-valinyl-D-lactyl-),
Cyclo(-N-methyl-L-alanyl-D-lactyl-N-methyl-L-isoleucyl-D-3-morpholino-phenyllactyl-N-methyl-L-valinyl-D-lactyl-),
Cyclo(-N-methyl-L-alanyl-D-lactyl-N-methyl-L-isoleucyl-D-4-morpholino-phenyllactyl-N-methyl-L-valinyl-D-lactyl-),
Cyclo(-N-methyl-L-alanyl-D-lactyl-N-methyl-L-isoleucyl-D-3-(2-hydroxyethylsulfonylethylamino-phenyl)lactyl-N-methyl-L-valinyl-D-lactyl-),
Cyclo(-N-methyl-L-alanyl-D-lactyl-N-methyl-L-isoleucyl-D-4-(2-hydroxyethylsulfonylethylamino-phenyl)lactyl-N-methyl-L-valinyl-D-lactyl-),
Cyclo(-N-methyl-L-alanyl-D-lactyl-N-methyl-L-valinyl-D-2-nitro-phenyllactyl-N-methyl-L-valinyl-D-lactyl-),
Cyclo(-N-methyl-L-alanyl-D-lactyl-N-methyl-L-valinyl-D-3-nitro-phenyllactyl-N-methyl-L-valinyl-D-lactyl-),
Cyclo(-N-methyl-L-alanyl-D-lactyl-N-methyl-L-valinyl-D-4-nitro-phenyllactyl-N-methyl-L-valinyl-D-lactyl-),
Cyclo(-N-methyl-L-alanyl-D-lactyl-N-methyl-L-valinyl-D-2-amino-phenyllactyl-N-methyl-L-valinyl-D-lactyl-),
Cyclo(-N-methyl-L-alanyl-D-lactyl-N-methyl-L-valinyl-D-3-amino-phenyllactyl-N-methyl-L-valinyl-D-lactyl-),
Cyclo(-N-methyl-L-alanyl-D-lactyl-N-methyl-L-valinyl-D-4-amino-phenyllactyl-N-methyl-L-valinyl-D-lactyl-),
Cyclo(-N-methyl-L-alanyl-D-lactyl-N-methyl-L-valinyl-D-3-morpholino-phenyllactyl-N-methyl-L-valinyl-D-lactyl-),
Cyclo(-N-methyl-L-alanyl-D-lactyl-N-methyl-L-valinyl-D-4-morpholino-phenyllactyl-N-methyl-L-valinyl-D-lactyl-),
Cyclo(-N-methyl-L-alanyl-D-lactyl-N-methyl-L-valinyl-D-3-(2-hydroxyethylsulfonyl-ethylaminophenyl)lactyl-N-methyl-L-valinyl-D-lactyl-),
Cyclo(-N-methyl-L-alanyl-D-lactyl-N-methyl-L-valinyl-D-4-(2-hydroxyethylsulfonyl-ethylaminophenyl)lactyl-N-methyl-L-valinyl-D-lactyl-),
Cyclo(-N-methyl-L-alanyl-D-lactyl-N-methyl-L-isoleucyl-D-2-nitro-phenyllactyl-N-methyl-L-alanyl-D-lactyl-),
Cyclo(-N-methyl-L-alanyl-D-lactyl-N-methyl-L-isoleucyl-D-3-nitro-phenyllactyl-N-methyl-L-alanyl-D-lactyl-),
Cyclo(-N-methyl-L-alanyl-D-lactyl-N-methyl-L-isoleucyl-D-4-nitro-phenyllactyl-N-methyl-L-alanyl-D-lactyl-),
Cyclo(-N-methyl-L-alanyl-D-lactyl-N-methyl-L-isoleucyl-D-2-amino-phenyllactyl-N-methyl-L-alanyl-D-lactyl-),
Cyclo(-N-methyl-L-alanyl-D-lactyl-N-methyl-L-isoleucyl-D-3-amino-phenyllactyl-N-methyl-L-alanyl-D-lactyl-),
Cyclo(-N-methyl-L-alanyl-D-lactyl-N-methyl-L-isoleucyl-D-4-amino-phenyllactyl-N-methyl-L-alanyl-D-lactyl-),
Cyclo(-N-methyl-L-alanyl-D-lactyl-N-methyl-L-isoleucyl-D-3-morpholino-phenyllactyl-N-methyl-L-alanyl-D-lactyl-),
Cyclo(-N-methyl-L-alanyl-D-lactyl-N-methyl-L-isoleucyl-D-4-morpholino-phenyllactyl-N-methyl-L-alanyl-D-lactyl-),
Cyclo(-N-methyl-L-alanyl-D-lactyl-N-methyl-L-isoleucyl-D-3-(2-hydroxyethylsulfonylethylamino-phenyl)lactyl-N-methyl-L-alanyl-D-lactyl-),
Cyclo(-N-methyl-L-alanyl-D-lactyl-N-methyl-L-isoleucyl-D-4-(2-hydroxyethylsulfonylethylamino-phenyl)lactyl-N-methyl-L-alanyl-D-lactyl-),
Cyclo(-N-methyl-L-alanyl-D-lactyl-N-methyl-L-isoleucyl-D-2-nitro-phenyllactyl-N-methyl-L-2-amino-butyl-D-lactyl-),
Cyclo(-N-methyl-L-alanyl-D-lactyl-N-methyl-L-isoleucyl-D-3-nitro-phenyllactyl-N-methyl-L-2-amino-butyl-D-lactyl-),
Cyclo(-N-methyl-L-alanyl-D-lactyl-N-methyl-L-isoleucyl-D-4-nitro-phenyllactyl-N-methyl-L-2-amino-butyl-D-lactyl-),
Cyclo(-N-methyl-L-alanyl-D-lactyl-N-methyl-L-isoleucyl-D-2-amino-phenyllactyl-N-methyl-L-2-amino-butyl-D-lactyl-),
Cyclo(-N-methyl-L-alanyl-D-lactyl-N-methyl-L-isoleucyl-D-3-amino-phenyllactyl-N-methyl-L-2-amino-butyl-D-lactyl-),
Cyclo(-N-methyl-L-alanyl-D-lactyl-N-methyl-L-isoleucyl-D-4-amino-phenyllactyl-N-methyl-L-2-amino-butyl-D-lactyl-),
Gyclo(-N-methyl-L-alanyl-D-lactyl-N-methyl-L-isoleucyl-D-3-morpholino-phenyllactyl-N-methyl-L-2-amino-butyl-D-lactyl-),
Cyclo(-N-methyl-L-alanyl-D-lactyl-N-methyl-L-isoleucyl-D-4-morpholino-phenyllactyl-N-methyl-L-2-amino-butyl-D-lactyl-),
Cyclo(-N-methyl-L-alanyl-D-lactyl-N-methyl-L-isoleucyl-D-3-(2-hydroxyethylsulfonylethylamino-phenyl)lactyl-N-methyl-L-2-amino-butyl-D-lactyl-),
Cyclo(-N-methyl-L-alanyl-D-lactyl-N-methyl-L-isoleucyl-D-4-(2-hydroxyethylsulfonylethylamino-phenyl)lactyl-N-methyl-L-2-amino-butyl-D-lactyl-),
Cyclo(-N-methyl-L-alanyl-D-lactyl-N-methyl-L-isoleucyl-D-2-nitro-phenyllactyl-N-methyl-L-leucyl-D-lactyl-),
Cyclo(-N-methyl-L-alanyl-D-lactyl-N-methyl-L-isoleucyl-D-3-nitro-phenyllactyl-N-methyl-L-leucyl-D-lactyl-),
Cyclo(-N-methyl-L-alanyl-D-lactyl-N-methyl-L-isoleucyl-D-4-nitro-phenyllactyl-N-methyl-L-leucyl-D-lactyl-),
Cyclo(-N-methyl-L-alanyl-D-lactyl-N-methyl-L-isoleucyl-D-2-amino-phenyllactyl-N-methyl-L-leucyl-D-lactyl-),
Cyclo(-N-methyl-L-alanyl-D-lactyl-N-methyl-L-isoleucyl-D-3-amino-phenyllactyl-N-methyl-L-leucyl-D-lactyl-),
Cyclo(-N-methyl-L-alanyl-D-lactyl-N-methyl-L-isoleucyl-D-4-amino-phenyllactyl-N-methyl-L-leucyl-D-lactyl-),
Cyclo(-N-methyl-L-alanyl-D-lactyl-N-methyl-L-isoleucyl-D-3-morpholino-phenyllactyl-N-methyl-L-leucyl-D-lactyl-),
Cyclo(-N-methyl-L-alanyl-D-lactyl-N-methyl-L-isoleucyl-D-4-morpholino-phenyllactyl-N-methyl-L-leucyl-D-lactyl-),
Cyclo(-N-methyl-L-alanyl-D-lactyl-N-methyl-L-isoleucyl-D-3-(2-hydroxyethylsulfonylethylamino-phenyl)lactyl-N-methyl-L-leucyl-D-lactyl-),
Cyclo(-N-methyl-L-alanyl-D-lactyl-N-methyl-L-isoleucyl-D-4-(2-hydroxyethylsulfonylethylamino-phenyl)lactyl-N-methyl-L-leucyl-D-lactyl-).

Die gegebenenfalls substituierten Reste der allgemeinen Formeln können einen oder mehrere, vorzugsweise 1 bis 3, insbesondere 1 bis 2 gleiche oder verschiedene Substituenten tragen. Als Substituenten seien beispielhaft und vorzugsweise aufgeführt:

Alkyl mit vorzugsweise 1 bis 4, insbesondere 1 bis 2 Kohlenstoffatomen, wie Methyl, Ethyl, n-Propyl, Isopropyl, n-Butyl, Isobutyl, sec-Butyl und tert-Butyl; Alkoxy mit vorzugsweise 1 bis 4, insbesondere 1 bis 2 Kohlenstoffatomen, wie Methoxy, Ethoxy, n-Propoxy, Isopropoxy, n-Butoxy, Isobutoxy, sec-Butoxy und tert-Butoxy; Alkylthio wie Methylthio, Ethylthio, n-Propylthio, Isopropylthio, n-Butylthio, Isobutylthio, sec-Butylthio; Halogenalkyl mit vorzugsweise 1 bis 5, insbesondere 1 bis 3 Halogenatomen, wobei die Halogenatome gleich oder verschieden sind und Halogenatome, vorzugsweise für Fluor, Chlor oder Brom, insbesondere Fluor oder Chlor stehen, wie Difluormethyl, Trifluormethyl, Trichlormethyl; Hydroxy; Halogen, vorzugsweise Fluor, Chlor, Brom und Iod, insbesondere Fluor und Chlor; Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methylethylamino, Dimethylamino, n-Propylamino, Isopropylamino, Methyl-n-butylamino; Alkylcarbonylreste wie Methylcarbonyl; Alkoxycarbonyl mit vorzugsweise 2 bis 4, insbesondere 2 bis 3 Kohlenstoffatomen wie Methoxycarbonyl und Ethoxycarbonyl; Alkylsulfinyl mit 1 bis 4, insbesondere 1 bis 2 Kohlenstoffatomen; Halogenalkylsulfinyl mit 1 bis 4, insbesondere 1 bis 2 Kohlenstoffatomen und 1 bis 5 Halogenatomen wie Trifluormethylsulfinyl; Halogenalkylsulfonyl mit 1 bis 4, insbesondere 1 bis 2 Kohlenstoffatomen und 1 bis 5 Halogenatomen wie Trifluormethylsulfonyl, Perfluor-n-butylsulfonyl, Perfluor-isobutylsulfonyl; Arylsulfonyl mit vorzugsweise 6 oder 10 Arylkohlenstoffatomen wie Phenylsulfonyl; Acyl, Aryl, Aryloxy, die ihrerseits einen der oben genannten Substituenten sowie den Formiminorest (-HC=N-O-Alkyl) tragen können.

Die Verbindungen der allgemeinen Formel (I) sind neu, sie lassen sich beispielsweise nach dem oben angegebenen Verfahren herstellen.

Nachfolgend wird das erfindungsgemäße Verfahren anhand ausgewählter Beispiele erläutert (vgl. auch Herstellungsbeispiele).

Setzt man beispielsweise bei Verfahren 2a zur Nitrierung das cyclische Depsipeptid Cyclo(-N-methyl-L-alanyl-D-lactyl-N-methyl-L-isoleucyl-D-phenyllactyl-N-methyl-L-isoleucyl-D-lactyl-) als Verbindung der allgemeinen Formel (II) ein und verwendet als Nitrierungsmittel rauchende Salpetersäure, so entsteht ein Gemisch aus Cyclo(-N-methyl-L-alanyl-D-lactyl-N-methyl-L-isoleucyl-D-2-nitro-phenyllactyl-N-methyl-L-isoleucyl-D-lactyl-), Cyclo(-N-methyl-L-alanyl-D-lactyl-N-methyl-L-isoleucyl-D-3-nitro-phenyllactyl-N-methyl-L-isoleucyl-D-lactyl-) und Cyclo(-N-methyl-L-alanyl-D-laetyl-N-methyl-L-isoleucyl-D-4-nitro-phenyllactyl-N-methyl-L-isoleucyl-D-lactyl-) (vgl. Schema 1).

Die zur Durchführung des Verfahrens 2a als Ausgangsstoffe benötigten Verbindungen sind durch die Formeln (II) allgemein definiert. In den Formeln (II) stehen vorzugsweise für R¹ bis R⁴ diejenigen Reste, die bereits im Zusammenhang mit der Beschreibung der erfindungsgemäßen Stoffe der Formel ( I ) als bevorzugt für diese Substituenten genannt wurden.

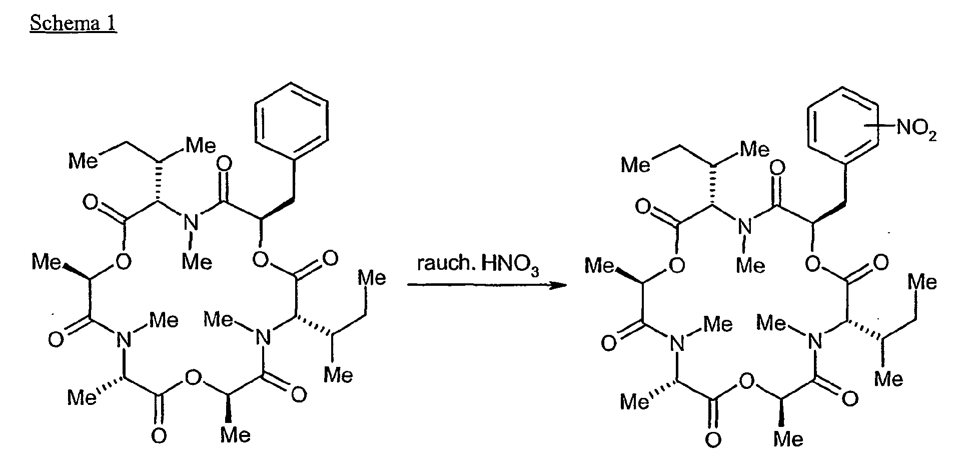

Die als Ausgangsstoffe verwendeten cyclischen Depsipeptide sind z. T. bekannt und können nach literaturbekannten Methoden mittels Totalsynthese (DE 4317458 A1 EP 658551 A1)

Beispielsweise können die als Ausgangsstoffe verwendeten cyclischen Depsipeptide der allgemeinen Formel (II) durch Cyclisierung aus entsprechenden offenkettigen Hexadepsipeptiden (z. B. DE 4317458, EP 658551 A1; Jeschke et al. Bioorg. Chem. 1999, S. 207-214), die beispielsweise nach literaturbekannten Methoden darstellbar sind (z. B. JP 07196486 A2; offenkettige Tetradepsipeptide: JP 07196487 A2), erhalten werden (vgl. Schema 2).

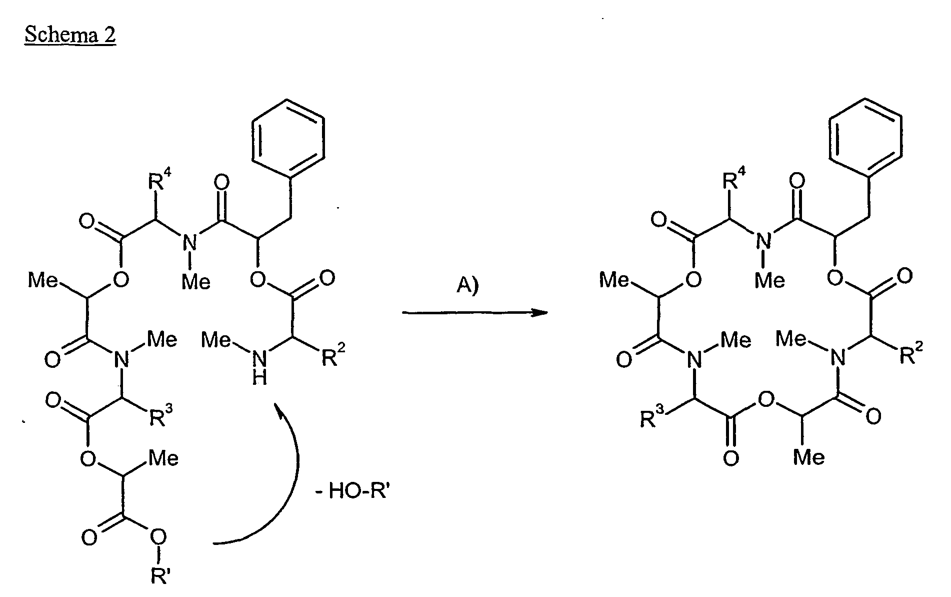

### A): BOP-C1, iPr₂N-Et, DCM, 0-25 °C, [48 h]; R' = H

Eine Cyclisierung der entsprechenden offenkettigen Hexadepsipeptiden gelingt beispielsweise bei Verwendung eines Aktivesters (R' = Pentafluorphenyl) (vgl. auch Verfahren zur Darstellung von makrocyclischen Peptidalkaloiden: U. Schmidt et al. In Synthesis 1991, S. 294-300 [Didemnin A, B und C]; Angew. Chem. 96, 1984, S. 723-724 [Dolastin 3]; Angew. Chem. 102, 1990, S. 562-563 [Fenestin A]) oder im Falle N,O-terminal debockierter Hexadepsipeptide (R' = H) bevorzugt in . Gegenwart von Kupplungsreagenzien (vgl. z. B. Jeschke et al. Bioorg. Chem. 1999, S. 207-214).

Als Kupplungsreagenzien zur Cyclisierung der offenkettigen Hexadepsipeptide finden alle, die zur Herstellung einer Amidbindung geeignet sind (vgl. z. B. Houbel-Weyl, Methoden der organischen Chemie, Band 15/2; Bodansky et al., Peptide Synthesis 2nd ed. (Wiley & Sons, New York 1976) oder Gross, Meienhofer, The Peptides: Analysis, Synthesis, Biology (Academy Press, New York 1979), Verwendung. Vorzugsweise werden folgende Methoden herangezogen: Aktivestermethode mit Pentachlor- (Pcp) und Pentafluorphenol (Pfp), N-Hydroxysuccinimid (HOSu), N-Hydroxy-5-norbornen-2,3-dicarboxamid (HONB), 1-Hydroxy-benzotriazol (HOBt) oder 3-Hydroxy-4-oxo-3,4-dihydro-1,2,3-benzotriazin als Alkoholkomponente, Kupplung mit Carbodiimiden wie Dicyclohexylcarbodiimid (DCCI) nach dem DCC-Additiv-Verfahren, oder mit n-Propanphos-phosphonsäureanhydrid (PPA) und Gemischt-Anhydrid-Methode mit Pivaloylchlorid, Ethyl- (EEDQ) und Isobutyl-Chloroformiat (IIDQ) oder Kupplung mit Phosphoniumreagenzien, wie Benzotriazol-1-yloxy-tris(dimethylamino-phosphonium)-hexafluorophosphat (BOP), Bis(2-oxo-3-oxazolidinyl)phosphoniumsäurechlorid (BOP-Cl), Benzotriazol-1-yl-tris-pyrrolidino-phosphonium-hexafluorophosphat (PyBOB^{®}), Bromo-tris-pyrrolidino-phosphonium-hexafluorophospha (PyBroP^{®}) oder mit Phosphonsäureesterreagenzien, wie Cyanphosphonsäurediethylester (DEPC) und Diphenylphosphorylazid (DPPA) oder Uroniumreagenzien, wie 2-(1H-Benzotriazol-1-yl)-1,1,3,3-tetra-methyluronium-tetra-fluoroborat (TBTU), 2-(5-Norbornen-2,3-dicarboxamido)-1,1,3,3,-tetramethyl-uroniumtetrafluoroborat (TNTU), 2-(2-Oxo-1(2H)-pyridyl-1,1,3,3-bis-pentamethylen-tetramethyluroniumtetrafluoroborat (TSTU) oder wie 2-(1H-Benzotriazol-1-yl)-1,1,3,3-tetramethyluroniumhexafluorophosphat (HBTU).

Bevorzugt ist die Kupplung mit Phosphoniumreagenzien wie Bis(2-oxo-3-oxazolidinyl)phosphoniumsäurechlorid (BOP-Cl), Benzotriazol-1-yl-oxy-tris(dimethylamino-phosphonium)-hexafluorophosphat (BOP), Benzotriazol-1-yl-tris-pyrrolidino-phosphonium-hexafluorophosphat (PyBOB^{®}), Bromo-tris-pyrrolidino-phosphonium-hexafluorophospha (PyBroP^{®}) oder mit Phosphonsäureesterreagenzien, wie Cyanphosphonsäurediethylester (DEPC) und Diphenylphosphorylazid (DPPA).

Als basische Reaktionshilfsmittel zur Durchführung der Cyclisierung offenkettiger Hexadepsipeptide können alle geeigneten basischen Reaktionshilfsmittel eingesetzt werden wie Amine, insbesondere tertiäre Amine sowie Alkali- und Erdalkaliverbindungen.

Beispielhaft seien dafür erwähnt die Hydroxide, Oxide und Carbonate des Lithiums, Natriums, Kaliums, Magnesiums, Calciums und Bariums, ferner weitere basische Verbindungen wie Amidinbasen oder Guanidinbasen wie 7-Methyl-1,5,7-triazabicyclo(4.4.0)-dec-5-en (MTBD); Diazabicyclo(4.3.0)nonen (DBN), Diazabicyclo(2.2.2)-octan (DABCO), 1,8-Diaza-bicyclo(5.4.0)-undecen (DBU), Cyclohexyl-tetrabutylguanidin (CyTBG), Cyclohexyltetramethylguanidin (CyTMG), N,N,N,N-Tetramethyl-1,8-naphthalindiamin, Pentamethylpiperidin, tertiäre Amine wie Triethylamin, Trimethylamin, Tribenzylamin, Triisopropylamin, Tributylamin, Tribenzylamin, Tricyclohexylamin, Triamylamin, Trihexylamin, N,N-Dimethylanilin, N,N-Dimethyl-toluidin, N,N-Dimethyl-p-aminopyridin, N-Methyl-pyrrolidin, N-Methyl-piperidin, N-Methyl-imidazol, N-Methyl-pyrrol, N-Methyl-morpholin, N-Methyl-hexamethylenimin, Pyridin, 4-Pyrrolidinopyridin, 4-Dimethylamino-pyridin, Chinolin, α-Picolin, β-Picolin, Isochinolin, Pyrimidin, Acridin, N,N,N',N'-Tetramethylendiamin, N,N',N'-Tetraethylen-diamin, Chinoxalin, N-Propyl-diisopropylamin, N-Ethyl-diisopropylamin, N,N'-Dimethylcyclohexylamin, 2,6-Lutidin, 2,4-Lutidin oder Triethylendiamin.

Vorzugsweise finden tertiäre Amine, insbesondere Trialkylamine wie Triethylamin, N,N-Diisopropylethylamin, N-Propyl-diisopropylamin, N,N'-Dimethyl-cyclohexylamin oder N-Methyl-morpholin, Verwendung.

Nitrierungen sind nach üblichen Verfahren, wie sie beispielsweise in Houben-Weyl, Methoden der Organischen Chemie, Band XI/2 (Georg Thieme Verlag Stuttgart 1958), S. 99-116 beschrieben sind, durchführbar. Als Nitrierungsreagenzien seien rauchende oder 100 %ige Salpetersäure (Darstellung wasserfreier Salpetersäure vgl. F. D. Chattaway, Soc. 1910, 97, S. 2100) gegebenenfalls in Gegenwart von Schwefelsäure (M. J. Middleton et al., J. Heterocyclic Chem. 1970, 7, S. 1045-1049; L. W. Deady et al. Aust. J. Chem. 1982, 35 (10), S. 2025-2034; EP 0 192 060) oder die Verwendung von Salpetersäureestern, Acylnitrat oder Nitroniumtetrafluoroborat genannt..

Zur Durchführung des erfindungsgemäßen Verfahrens 2a werden als Nitrierungsagenzien bevorzugt rauchende oder 98-100 %ige Salpetersäure eingesetzt.

Die Nitrierung nach Verfahren 2a wird durchgeführt, indem man die Depsipeptide der allgemeinen Formel (II) in Gegenwart eines geeigneten Nitrierungsmittels, beispielsweise rauchende Salpetersäure, umsetzt.

Die Reaktionsdauer beträgt 5 Minuten bis 72 Stunden. Die Umsetzung erfolgt bei Temperaturen zwischen -50°C und 50°C, bevorzugt zwischen -30°C und 30°C, besonders bevorzugt bei Temperaturen zwischen -15°C und 15°C. Es kann grundsätzlich unter Normaldruck gearbeitet werden. Vorzugsweise arbeitet man bei Normaldruck oder bei Drucken bis zu 15 bar und gegebenenfalls unter Schutzgasatmosphäre (Stickstoff oder Helium).

Zur Durchführung des erfindungsgemäßen Verfahrens 2a setzt man pro mMol zu nitrierendes Depsipeptid 5 bis 10 ml, vorzugsweise 6 bis ml an Nitrierungsagenz ein.

Nach vollendeter Nitrierung wird der gesamte Reaktionsansatz neutralisiert, verdünnt und mit einem geeigneten organischen Lösungsmittel, beispielsweise Essigsäureethylester, extrahiert. Nach Abtrennen des organischen Lösungsmittels und Einengen im Vakuum, lassen sich die anfallenden Produkte in üblicher Weise durch Umkristallisieren, Vakuumdestillation oder Säulenchromatographie reinigen (vgl. Herstellungsbeispiele).

Setzt man anschließend beispielsweise bei Verfahrens 2b zur Reduktion das Gemisch aus Cyclo(-N-methyl-L-alanyl-D-lactyl-N-methyl-L-isoleucyl-D-2-nitro-phenyl-lactyl-N-methyl-L-isoleucyl-D-lactyl-), Cyclo(-N-methyl-L-alanyl-D-lactyl-N-methyl-L-isoleucyl-D-3-nitro-phenyllactyl-N-methyl-L-isoleucyl-D-lactyl-) und Cyclo(-N-methyl-L-alanyl-D-lactyl-N-methyl-L-isoleucyl-D-4-nitro-phenyllactyl-N-methyl-L-isoleucyl-D-lactyl-) als Verbindungen der allgemeinen Formel ( III ) ein und verwendet als Reduktionsmittel Wasserstoff in Gegenwart eines geeigneten Katalysators, beispielsweise Palladiumhydroxid-Kohle, so entsteht ein Gemisch aus Cyclo(-N-methyl-L-alanyl-D-lactyl-N-methyl-L-isoleucyl-D-2-amino-phenyllactyl-N-methyl-L-isoleucyl-D-lactyl-), Cyclo(-N-methyl-L-alanyl-D-lactyl-N-methyl-L-isoleucyl-D-3-amino-phenyllactyl-N-methyl-L-isoleucyl-D-lactyl-) und Cyclo(-N-methyl-L-alanyl-D-lactyl-N-methyl-L-isoleucyl-D-4-amino-phenyllactyl-N-methyl-L-isoleucyl-D-lactyl-). (vgl. Schema 3).

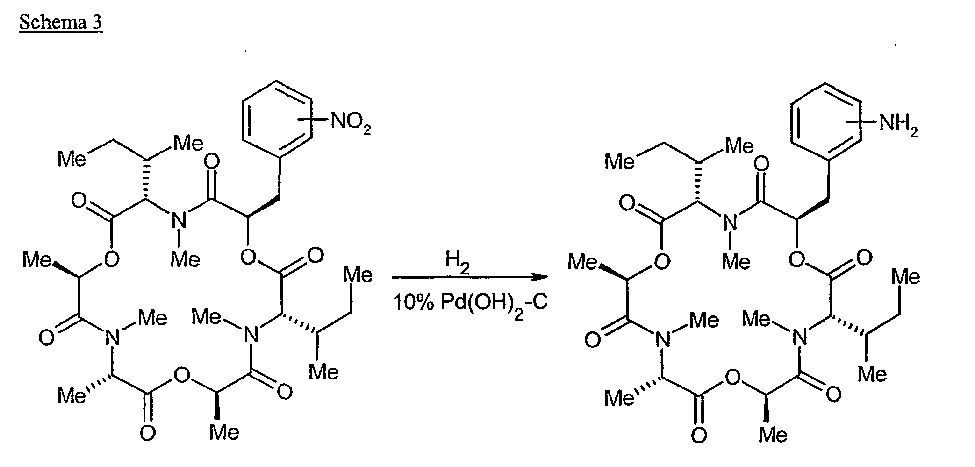

Die zur Durchführung des Verfahrens 2b als Ausgangsstoffe benötigten Verbindungen sind durch die Formeln (III) allgemein definiert. In den Formeln ( III ) stehen vorzugsweise für R¹ bis R⁴ diejenigen Reste, die bereits im Zusammenhang mit der Beschreibung der erfindungsgemäßen Stoffe der Formel (I) als bevorzugt für diese Substituenten genannt wurden.

Erfindungsgemäß und besonders bevorzugt ist die Hydrogenolyse von cyclischen Depsipeptiden der allgemeinen Formel (II) in Gegenwart eines Hydrierkatalysators.

Als geeignete Katalysatoren zur Durchführung der katalytischen Hydrierung kommen alle üblichen Hydrierkatalysatoren, wie beispielsweise Platin-Katalysatoren (Platin-Platte, Platin-Schwamm, Platin-Schwarz, kolloidales Platin, Platinoxid, Platindraht usw.), Palladium-Katalysatoren (beispielsweise Palladium-Schwamm, Palladium-Schwarz, Palladiumoxid, Palladium-Kohle, kolloidales Palladium, Palladium-Bariumsulfat, Palladium-Bariumkarbonat, Palladium-Hydroxid usw.), Ruthenium-Katalysatoren, Cobalt-Katalysatoren (beispielsweise reduziertes Cobalt, Raney Cobalt usw.), Kupfer-Katalysatoren (beispielsweise reduziertes Kupfer, Raney-Kupfer, Ullman-Kupfer usw.) in Frage. Vorzugsweise verwendet man jedoch Edelmetall-Katalysatoren, wie beispielsweise Platin- und Palladium- oder Ruthenium-Katalysatoren gegebenenfalls auf einem geeigneten Träger wie beispielsweise auf Kohlenstoff oder Silizium.

Als Hydrierkatalysatoren finden vorzugsweise Palladium-Katalysatoren, insbesondere Palladium- oder Palladium-Hydroxyd-Kohle, Verwendung.

Im allgemeinen ist es vorteilhaft, das erfindungsgemäße Verfahren 2a in Gegenwart von Verdünnungsmitteln durchzuführen. Verdünnungsmittel werden vorteilhaft in einer solcher Menge eingesetzt, dass das Reaktionsgemisch während des ganzen Verfahrens gut rührbar bleibt. Als Verdünnungsmittel zur Durchführung des erfindungsgemäßen Verfahrens kommen alle inerten Lösungsmittel in Frage.

Als Beispiele sind zu nennen: Halogenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlokohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Alkohole wie Methanol, Ethanol, Isopropanol, Butanol; Ether wie Ethylpropylether, Methyl-tert-butylether, n-Butylether, Anisol, Phenetol, Cyclohexylmethylether, Dimethylether, Diethylether, Dipropylether, Diisopropylether, Di-n-propylether, Diisopropylether, Diisobutylether, Diisoamylether, Ethylenglycoldimethylether, Tetrahydrofuran, Dioxan, Dichlodiethylether und Polyether des Ethylenoxids und/oder Propylenoxids; Amine wie Trimethyl-, Triethyl-, tripropyl-, Tributylamin, N-Methyl-morpholin, Pyridin und Tetramethylendiamin, Nitrokohlenwasserstoffe wie Nitromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril sowie Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylhexyl-, Ethylpropyl-, Ethylisobutyl- und Pentamethylensulfon; aliphatische, cycloaliphatische oder aromatische Kohlenwasserstoffe, beispielsweise sogenannte White Spirits mit Komponenten mit Siedepunkten im Bereich beispielsweise von 40°C bis 250°C, Cymol, Benzinfraktionen innerhalb eines Siedepunktintervalls von 70°C bis 190°C, Cyclohexan, Methylcyclohexan, Petrolether, Ligroin, Octan, Benzol, Toluol, Chlorbenzol, Brombenzol, Nitrotoluol, Xylol; Ester wie Methyl-, Ethyl-, Butyl-, Isobutylacetat, sowie Dimethyl-, Dibutyl-, Ethylencarbonat; Amide wie Hexamethylenphosphorsäuretriamid, Formamid, N-Methyl-formamid N,N-Dimethylformamid, N,N-Dipropylformamid, N,N-Dibutylformamid, n-Methyl-pyrrolidin, N-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-formylpiperidin, N,N'-1,4-diformylpiperazin; Ketone wie Aceton, Acetophenon, Methyl-ethylketon, Methyl-butylketon.

Selbstverständlich kann man das erfindungsgemäße Verfahren auch in Gemischen der genannten Lösung- und Verdünnungsmittel durchführen.

Die zu verwendenden Verdünnungsmittel sind vom jeweils eingesetzten Reduktionsmittels abhängig.

Bevorzugte Verdünnungsmittel zur Reduktion sind jedoch Alkohole, wie beispielsweise Methanol oder Ethanol.

Die als Ausgangsstoffe verwendeten cyclischen Depsipeptide sind z. T. bekannt und können nach Verfahren 2a mittels Nitrierung erhalten werden.

Die Reduktion nach Verfahren 2b wird durchgeführt, indem man die Depsipeptide der allgemeinen Formel (III) in Gegenwart eines geeigneten Reduktionsmittels, beispielsweise Wasserstoff in Gegenwart des Katalysators Paladiumhydroxid-Kohle, umsetzt.

Die Reaktionsdauer beträgt 10 Minuten bis 72 Stunden. Die Umsetzung erfolgt bei Temperaturen zwischen -20°C und 50°C, bevorzugt zwischen -10°C und 30°C, besonders bevorzugt bei Temperaturen zwischen -5°C und 10°C. Es kann grundsätzlich unter Normaldruck gearbeitet werden. Vorzugsweise arbeitet man bei Normaldruck oder bei Drucken bis zu 15 bar und gegebenenfalls unter Schutzgasatmosphäre (Stickstoff oder Helium).

Zur Durchführung des erfindungsgemäßen Verfahrens 2b setzt man pro mMol zu reduzierendes Depsipeptid, vorzugsweise 0,05 bis 1,5 g an Reduktionsmittel ein.

Nach vollendeter Reduktion wird das Reduktionsmittel abgetrennt und der gesamte Reaktionsansatz im Vakuum eingeengt. Die anfallenden Produkte lassen sich in üblicher Weise durch Umkristallisieren, Vakuumdestillation oder Säulenchromatographie reinigen (vgl. Herstellungsbeispiele).

Setzt man anschließend beispielsweise bei Verfahrens 2c zur Aminoalkylierung das reine Cyclo(-N-methyl-L-alanyl-D-lactyl-N-methyl-L-isoleucyl-D-4-amino-phenyl-lactyl-N-methyl-L-isoleucyl-D-lactyl-) als Verbindungen der allgemeinen Formel ( III ) ein und verwendet als Aldehyd einen geeigneten Dialdehyd, z. B. den in-situ erzeugten HOC-CH₂-O-CH₂-CHO, so entsteht das Cyclo(-N-methyl-L-alanyl-D-lactyl-N-methyl-L-isoleucyl-D-4-morpholino-phenyllactyl-N-methyl-L-isoleucyl-D-lactyl-) (vgl. Schema 4).

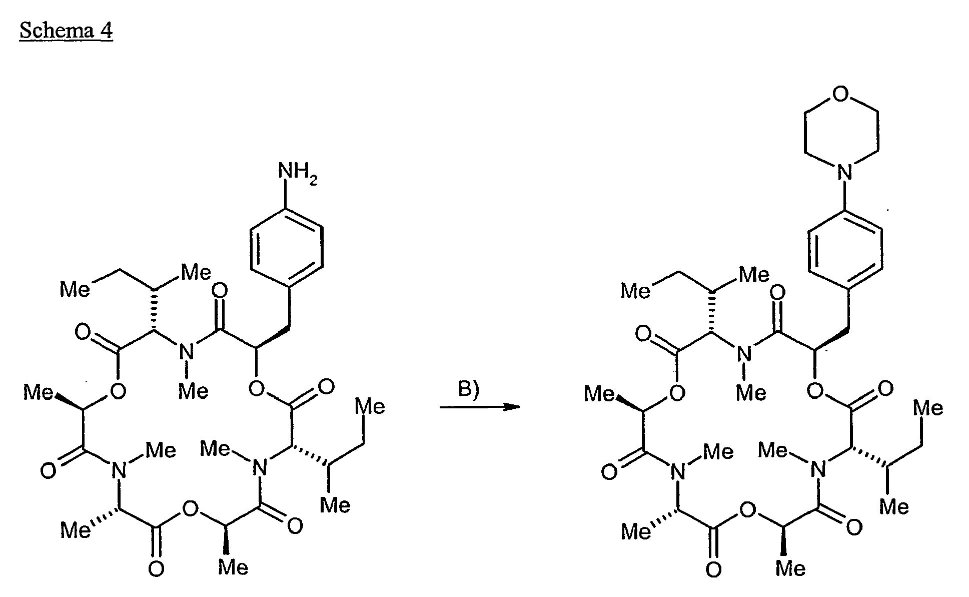

### B) in-situ [OHC-CH₂-O-CH₂-CHO], NaCNBH₃, Methanol, -50°C

Die zur Durchführung des Verfahrens 2c als Ausgangsstoffe benötigten Verbindungen sind durch die Formeln (IV) allgemein definiert. In den Formeln ( IV ) stehen vorzugsweise für R¹ bis R⁴ diejenigen Reste, die bereits im Zusammenhang mit der Beschreibung der erfindungsgemäßen Stoffe der Formel ( I ) als bevorzugt für diese Substituenten genannt wurden.

Die als Ausgangsstoffe verwendeten cyclischen Depsipeptide können nach Verfahren 2b erhalten werden.

Der als Ausgangsstoff eingesetzte Dialdehyd kann nach literaturbekannten Verfahren, beispielsweise (i) durch Natriumperiodat Oxidation aus 1,4-Anhydro-*meso*-erythrit (E. M. Acton et al. J. Med. Chem. 1984, 27, S. 638-645), (ii) durch Hydrolyse des Diketals (RO)₂-CH₂-O-CH₂-(O-R)₂ in Gegenwart von 50 %iger Essigsäure (F. J. lopez Aparicio et al. Carbohydrat Res. 1982, 111 (1), S. 157-162; WO 93/10053) oder (iii) mittels Ozonolyse aus 2,5-Dihydrofuran (X = O) (M. Kanemoto Chemistry Express 1987, 2 (1), S. 17-20), erhalten werden (vgl. Schema 5).

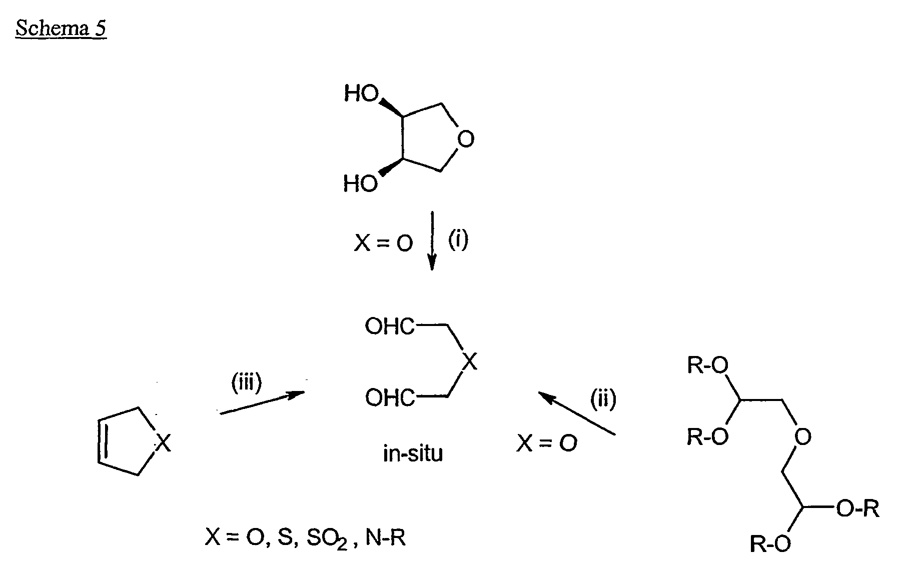

Eine Vielzahl unterschiedlicher Reduktionsmittel für reduktive Aminierungen sind in der Literatur beschrieben (vgl. Houben-Weyl XI/1, S. 602; W. S. Emerson, Org. Reactions 4, 1949, S. 174; E. M. Hancock, A. C. Cope Org. Synth., Coll. Vol. III, 1955, S. 717). Beispielsweise kommen für die Hydrierung der in-situ gebildeten Azomethine die verschiedensten Hydrierungsagenzien, wie beispielsweise Alkalimetallhydride, insbesondere Natriumborhydrid (NaBH₄), Natriumcyanoborhydrid (NaCNBH₃), Lithiumaluminiumhydrid (LiAlH₄), Lithiumtriethylborhydrid (Li[Et₃BH]), Lithium-tri-*sec*-borhydrid (Li[*sec*-Bu₃BH]), Natrium-*bis*(2-methoxyethoxy)aluminium-hydrid, Alkylaluminiumhydride, insbesondere Diisobutylaluminiumhydrid (DIBAL-H) oder Tetrametylammoniumtriacetoxyborhydrid u.a. in Frage (vgl. H. de Koning, W. N. Speckamp, Houben Weyl E 21, S. 1953 und dort zitierte Literatur).

Selbstverständlich kann auch ein "Borhydrid-Harz" beispielsweise "Borohydride on Amberlite^{®} IRA-406", zur Hydrierung verwendet werden (vgl. Sande A. R. et al., Tetrahedron Lett. 1984, 25, S.3501).

Zur Durchführung des erfindungsgemäßen Verfahrens 2c werden bevorzugt Alkalimetallhydride, insbesondere Natriumborhydrid (NaBH₄), Natriumcyanoborhydrid (NaCNBH₃), Lithiumaluminiumhydrid (LiAlH₄), eingesetzt.

Die reduktive Alkylierung nach Verfahren 2b wird durchgeführt, indem man die Depsipeptide der allgemeinen Formel (III) in Gegenwart eines Verdünnungsmittels und in Gegenwart eines Aldehyds und eines geeigneten Reduktionsmittels, beispielsweise Natriumcyanoborhydrid, umsetzt.

Die Reaktionsdauer beträgt 10 Minuten bis 72 Stunden. Die Umsetzung erfolgt bei Temperaturen zwischen -20°C und 50°C, bevorzugt zwischen -10°C und 30°C, besonders bevorzugt bei Temperaturen zwischen -5°C und 10°C. Es kann grundsätzlich unter Normaldruck gearbeitet werden. Vorzugsweise arbeitet man bei Normaldruck oder bei Drucken bis zu 15 bar und gegebenenfalls unter Schutzgasatmosphäre (Stickstoff oder Helium).

Zur Durchführung des erfindungsgemäßen Verfahrens 2c setzt man pro mMol Depsipeptid, vorzugsweise 1,0 mMol bis 3,0 mMol an Reduktionsmittel ein.

Nach vollendeter Aminoalkylierung wird der gesamte Reaktionsansatz neutralisiert, verdünnt und mit einem organischen Lösungsmittel, beispielsweise einem Chlorkohlenwasserstoff, extrahiert. Nach dem Waschen der organischen Phase, Trocknen und Einengen im Vakuum lassen sich die anfallenden Produkte in üblicher Weise durch Umkristallisieren, Vakuumdestillation oder Säulenchromatographie reinigen (vgl. Herstellungsbeispiele).

Setzt man in einer weiteren Ausführungsform des Verfahrens 2c zur Aminoalkylierung das reine Cyclo(-N-methyl-L-alanyl-D-lactyl-N-methyl-L-isoleucyl-D-4-amino-phenyl-lactyl-N-methyl-L-isoleucyl-D-lactyl-) als Verbindungen der allgemeinen Formel (III) ein und verwendet als Aldehyd einen Dialdehyd, beispielsweise HOC-CH₂-SO₂-CH₂-CHO ein, so entsteht überraschenderweise und erfindungsgemäß das Cyclo(-N-methyl-L-alanyl-D-lactyl-N-methyl-L-isoleucyl-D-4-(2-hydroxyethylsulfonyl-ethylamino-phenyl)lactyl-N-methyl-L-isoleucyl-D-lactyl-) (vgl. Schema 6).

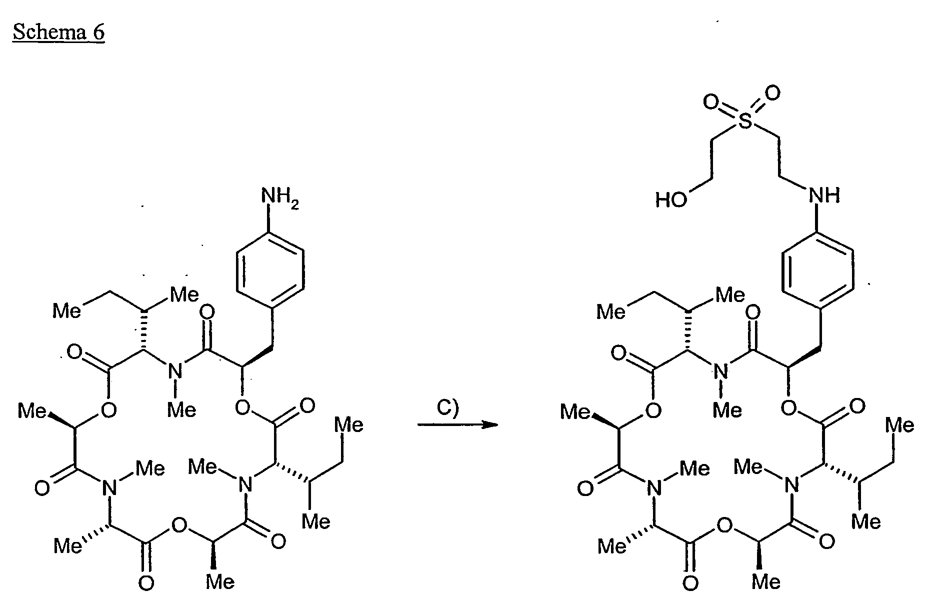

### C) in-situ [OHC-CH₂-SO₂-CH₂-CHO], NaCNBH₃, Methanol, -50°C

Die zur Durchführung des Verfahrens 2c als Ausgangsstoffe benötigten Verbindungen sind durch die Formeln (IV) allgemein definiert. In den Formeln (IV) stehen vorzugsweise für R¹ bis R⁴ diejenigen Reste, die bereits im Zusammenhang mit der Beschreibung der erfindungsgemäßen Stoffe der Formel ( I ) als bevorzugt für diese Substituenten genannt wurden.

Die als Ausgangsstoffe verwendeten cyclischen Depsipeptide können nach Verfahren 2b erhalten werden.

Der als Ausgangsstoff eingesetzte Dialdehyd kann nach dem literaturbekannten Verfahren (iii) in Schema 5 mittels Ozonolyse aus 3-Sulfolen (X = SO₂), erhalten werden.

Die reduktive Alkylierung nach Verfahren 2c wird durchgeführt, indem man die Depsipeptide der allgemeinen Formel ( III ) in Gegenwart eines Lösungsmittels und in Gegenwart eines Aldehyds und eines geeigneten Reduktionsmittels, beispielsweise Natriumcyanoborhydrid, umsetzt.

Die Reaktionsdauer beträgt 10 Minuten bis 72 Stunden. Die Umsetzung erfolgt bei Temperaturen zwischen -20°C und 50°C, bevorzugt zwischen -10°C und 30°C, besonders bevorzugt bei Temperaturen zwischen -5°C und 10°C. Es kann grundsätzlich unter Normaldruck gearbeitet werden. Vorzugsweise arbeitet man bei Normaldruck oder bei Drucken bis zu 15 bar und gegebenenfalls unter Schutzgasatmosphäre (Stickstoff oder Helium).

Zur Durchführung des erfindungsgemäßen Verfahrens 2c setzt man pro mMol Depsipeptid, vorzugsweise 1,0 bis 3,0 mMol an Reduktionsmittel ein.

Nach vollendeter Aminoalkylierung wird der gesamte Reaktionsansatz neutralisiert, verdünnt und mit einem organischen Lösungsmittel, beispielsweise einem Chlorkohlenwasserstoff, extrahiert. Nach dem Waschen der organischen Phase, Trocknen und Einengen im Vakuum lassen sich die anfallenden Produkte in üblicher Weise durch Umkristallisieren, Vakuumdestillation oder Säulenchromatographie reinigen (vgl. Herstellungsbeispiele).

Alternativ und in einer weiteren Ausführungsform kann die Ringschlussreaktion auch durch Umsetzung der Verbindungen der allgemeinen Formel (IV) mit Verbindungen der allgemeinen Formel (V) gegebenenfalls in Gegenwart eines weiter oben genannten basischen Reaktionshilfsmittels erfolgen:

E-CHR'₁-CHR'₂-X-CHR'₃-CHR'₄-E (V)

In den Verbindungen der allgemeinen Formel (V) steht X bevorzugt für Sauerstoff, Schwefel, Sulfonyl oder gegebenenfalls substituiertes Amino, die Reste R'1-R'4 stehen bevorzugt für C₁-C₂-Alkyl, beispielsweise Methyl oder Ethyl, E steht bevorzugt für eine geeignete Abgangsgruppe, beispielsweise Halogen, insbesondere Fluor, Chlor, Brom oder Iod, Methylsulfonyloxy (Ms-O) oder para-Toluolsulfonyloxy (p-Tos-O).

Die Verbindungen der allgemeinen Formel (V) sind literaturbekannt und deren Verwendung, beispielsweise für Ringschlussreaktionen ist beschrieben (vgl. WO 93/10053). Die Alkylierung wird beispielsweise durchgeführt, indem man die Depsipeptide der allgemeinen Formel (III) in Gegenwart eines Lösungsmittels und in Gegenwart eines basischen Reaktionshilfsmittels nach Schema 7 umsetzt.

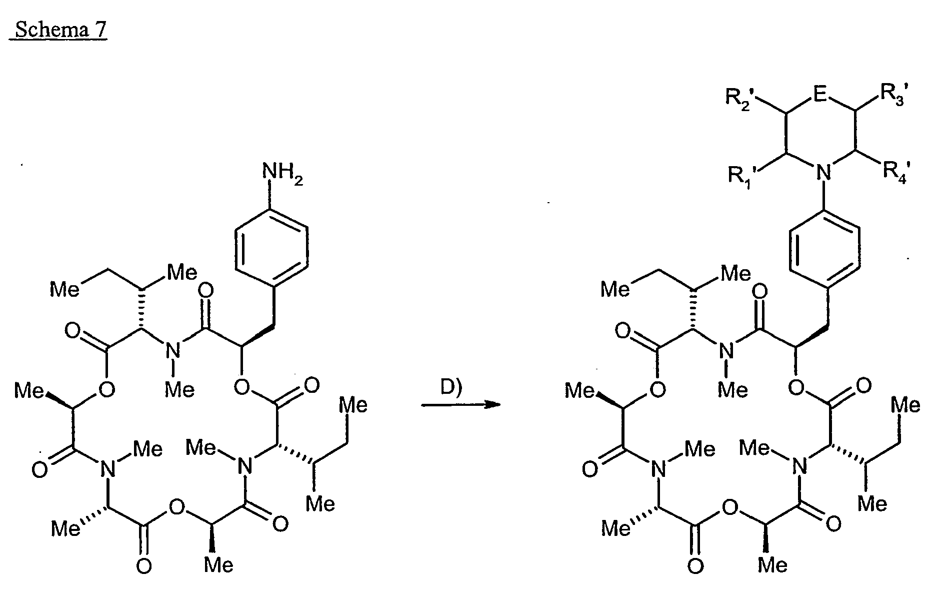

Mit dem Verfahren 2 sind, aus den einzelnen Bausteinen mit sowohl (S)- als auch (R)-Konfiguration (bzw. L- und D-Konfiguration), cyclische Depsipeptide unter Beibehaltung der ursprünglichen Konfiguration der Ausgangsstoffe erhältlich.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die Wirkstoffe eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Bevorzugt ist die Anwendung bei Endoparasiten von Warmblütern, insbesondere Säugetieren. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so dass durch den Einsatz der Wirkstoffe eine wirtschaftlichere und einfachere Tierhaltung möglich ist. Zu den pathogenen Endoparasiten zählen Cestoden, Trematoden, Nematoden, insbesondere:

Aus der Ordnung der Pseudophyllidea z.B.: Diphyllobothrium spp., Spirometra spp., Schistocephalus spp., Ligula spp., Bothridium spp., Diplogonorus spp..

Aus der Ordnung der Cyclophyllidea z.B.: Mesocestoides spp., Anoplocephala spp., Paranoplocephala spp., Moniezia spp., Thysanosma spp., Thysaniezia spp., Avitellina spp., Stilesia spp., Cittotaenia spp., Anhyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Omithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhloccelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonismus spp., Dicrocoelium spp., Collyriclum spp., Nanophyetus spp., Opisthorchis spp., Clonorchis spp., Metorchis spp., Heterophyes spp., Metagonimus spp..

Aus der Ordnung der Enoplida z.B.: Trichuris spp., Capillaria spp., Trichlomosoides spp., Trichinella spp..

Aus der Ordnung des Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostromum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostomum spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp., Cylicocyclus spp., Cylicodontophorus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Gruppe der Gigantohynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z. B. Streifen, Platten, Bänder, Halsbänder, Ohrmarken, Gliedmaßenbänder, Markierungsvornchtungen.

Die enterale Anwendung der Wirkstoffe geschieht z.B. oral in Form von Pulver, Zäpfchen, Tabletten, Kapseln, Fasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspensionen zur oralen oder dermalen Anwendung sowie zur Injektion; halbfeste Zubereitungen;

Formulierungen bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramusculär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht oder durch Tauchen (Dippen, Baden oder Waschen) aufgebracht. Diese Lösungen werden wie oben bei den Injektionslösungen beschrieben hergestellt.

Es kann vorteilhaft sein, bei der Herstellung Verdickungsmittel zuzufügen.

Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt, indem Lösungen, die wie bei den Injektionslösungen beschrieben hergestellt worden sind, mit soviel Verdickungsmittel versetzt werden, dass eine klare Masse mit salbenartiger Konsistenz entsteht. Als Verdickungsmittel werden die weiter oben angegebenen Verdickungsmittel eingesetzt.

Aufgießformulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgießformulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglykole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglycolmonomethylether, Diethylenglykolmono-butylether, Ketone wie Aceton, Methyl-ethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfat, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen können oral, dermal oder als Injektion angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase gelöst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-biglycerid, Triglyceridgemisch mit Pflanzenfettsäure der Kettenlänge C₈₋₁₂ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Cypryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a..

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglykol, Glycerin, Sorbitol und ihre Gemische.
Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien, Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben Angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker-, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Der erfindungsgemäße Wirkstoff kann in seinen Zubereitungen sowie in den aus diesen Zubereitungen bereiteten Anwendungsformen in Mischung mit anderen Wirkstoffen, wie Insektiziden, Sterilantien, Bakteriziden, Akariziden, Nematiziden oder Fungiziden vorliegen. Zu den Insektiziden zählen beispielsweise Phosphorsäureester, Carbamate, Carbonsäureester, chlorierte Kohlenwasserstoffe, Phenylharnstoffe, Nicotinyle, Neonicotinyle, durch Mikroorganismen hergestellte Stoffe u.a.

Besonders günstige Mischpartner sind z.B. die folgenden:

### Fungizide:

Aldimorph, Ampropylfos, Ampropylfos-Kalium, Andoprim, Anilazin, Azaconazol, Azoxystrobin, Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin,
Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-b-propyl-1H-1,2,4-triazol-1-ethanol,
α-(2,4-Dichlorphenyl)-β-methoxy-a-methyl-1H-1,2,4-triazol-l-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-a-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholin-hydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-bden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioate,
S-Methyl-1,2,3-benzothiadiazol-7-carbothioat,
spiro[2H]-1-Benzopyran-2,1'(3'H)-isobenzofuran]-3'-on,

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen, Chinolone wie Ciprofloxacin, Danofloxacin, Difloxacin, Enrofloxacin, Flumequine, Ibafloxacin, Marbofloxacin, Norfloxacin, Ofloxacin, Orbifloxacin, Premafloxacin, Sarafloxacin,

### Insektizide / Akarizide / Nematizide:

Abamectin, Acephate, Acetamiprid, Acrinathrin, Alanycarb, Aldicarb, Aldoxycarb, Alphacypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Cis-Resmethrin, Cispermethrin, Clocythrin, Cloethocarb, Clofentezine, Coumafos, Cyanophos, Cycloprene, Cycloprothrin, Cyfluthrin, Cyhalothrin, Cyhexatin, Cypermethrin, Cyromazine, Cythioat, Chlothianidin,
Deltamethrin, Demeton M, Demeton S, Demeton-S-methyl, Diafenthiuron, Diazinon, Dichlorvos, Dicyclanil, Diflubenzuron, Dimethoat, Dimethylvinphos, Diofenolan, Disulfoton, Docusat-sodium, Dofenapyn, Dinotefuran,
Eflusilanate, Emamectin, Empenthrin, Endosulfan, Eprinometin, Esfenvalerate, Ethiofencarb, Ethion, Ethiprole, Ethoprophos, Etofenprox, Etoxazole, Etrimfos,
Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenthion,Fenvalerate, Fipronil, Fluazinam, Fluazuron, Flubrocythrinate, Flucycloxuron, Flucythrinate, Flufenoxuron, Flumethrin, Flutenzine, Fluvalinate, Fonophos, Fosmethilan, Fosthiazate, Fubfenprox, Furathiocarb, Flupyrazofos,
Granuloseviren
Halofenozide, HCH, Heptenophos, Hexaflumuron, Hexythiazox, Hydroprene,
Imidacloprid, Indoxacarb, Isazofos, Isofenphos, Isoxathion, Ivermectin,
Kernpolyederviren,
Lambda-cyhalothrin, Lufenuron
Malathion, Mecarbam, Metaldehyd, Methamidophos, Metharhizium anisopliae, Metharhizium flavoviride, Methidathion, Methiocarb, Methomyl, Methoprene, Methoxyfenozide, Metolcarb, Metoxadiazone, Metrifonat, Mevinphos, Milbemectin, Monocrotophos, Moxidectin,
Naled, Nitenpyram, Nithiazine, Novaluron, NEEM,
Omethoat, Oxamyl, Oxydemethon M
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propoxur, Prothiofos, Prothoat, Pymetrozine, Pyraclofos, Pyresmethrin, Pyrethrum, Pyridaben, Pyridathion, Pyrimidifen, Pyriproxyfen, Protrifenbute,
Quinalphos,
Ribavirin
Salithion, Sebufos, Silafluofen, Spinosad, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Theta-cypermethrin, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb, Thiacloprid,
Vamidothion, Vaniliprole, Verticillium lecanii
YI 5302
Zeta-cypermethrin, Zolaprofos
(1R-cis)-[5-(Phenylmethyl)-3-furanyl]-methyl-3-[(dihydro-2-oxo-3(2H)-furanyliden)-methyl]-2,2-dimethylcyclopropancarboxylat
(3-Phenoxyphenyl)-methyl-2,2,3,3-tetramethylcyclopropanecarboxylat
1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol
2-(Acetlyoxy)-3-dodecyl-1,4-naphthalindion
2-Chlor-N-[[[4-(1-phenylethoxy)-phenyl]-amino]-carbonyl]-benzamid
2-Chlor-N-[[[4-(2,2-dichlor-1,1-difluorethoxy)-phenyl]-amino]-carbonyl]-benzamid
3-Methylphenyl-propylcarbamat
4-[4-(4-Ethoxyphenyl)-4-methylpentyl]-1-fluor-2-phenoxy-benzol
4-Chlor-2-(1,1-dimethylethyl)-5-[[2-(2,6-dimethyl-4-phenoxyphenoxy)ethyl]thio]-3(2H)-pyridazinon
4-Chlor-2-(2-chlor-2-methylpropyl)-5-[(6-iod-3-pyridinyl)methoxy]-3(2H)-pyridazinon
4-Chlor-5-[(6-chlor-3-pyridinyl)methoxy]-2-(3,4-dichlorphenyl)-3 (2H)-pyridazinon
Bacillus thuringiensis strain EG-2348
Benzoesäure [2-benzoyl-1-(1,1-dimethylethyl)-hydrazid
Butansäure 2,2-dimethyl-3-(2,4-dichlorphenyl)-2-oxo-1-oxaspiro[4.5]dec-3-en-4-yl-ester
[3-[(6-Chlor-3-pyridinyl)methyl]-2-thiazolidinyliden]-cyanamid
Dihydro-2-(nitromethylen)-2H-1,3-thiazine-3(4H)-carboxaldehyd
Ethyl-[2-[[1,6-dihydro-6-oxo-1-(phenylmethyl)-4-pyridazinyl]oxy]ethyl]-carbamat
N-(3,4,4-Trifluor-1-oxo-3-butenyl)-glycin
N-(4-Chlorphenyl)-3-[4-(difluormethoxy)phenyl]-4,5-dihydro-4-phenyl-1H-pyrazol-1-carboxamid
N-[(2-Chlor-5-thiazolyl)methyl]-N'-methyl-N"-nitro-guanidin
N-Methyl-N'-(1-methyl-2-propenyl)-1,2-hydrazindicarbothioamid
N-Methyl-N'-2-propenyl-1,2-hydrazindicarbothioamid
O,O-Diethyl-[2-(dipropylamino)-2-oxoethyl]-ethylphosphoramidothioat

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch welche die Wirkung der Wirkstoffe gesteigert wird, ohne dass der zugesetzte Synergist selbst aktiv wirksam sein muß.

Anwendungsfertig/e Zubereitungen enthalten den Wirkstoff in Konzentrationen von 10 ppm bis 20 Gew.-%, bevorzugt von 0,1 bis 10 Gew.-%.

Zubereitungen die vor Anwendung verdünnt werden, enthalten den Wirkstoff in Konzentrationen von 0,5 bis 90 Gew.-%, bevorzugt von 5 bis 50 Gew.-%.

Im allgemeinen hat es sich als vorteilhaft erwiesen, Mengen von etwa 1 bis 100 mg Wirkstoff je kg Körpergewicht pro Tag zur Erzielung wirksamer Ergebnisse zu verabreichen.

### Beispiele

### Herstellungsbeispiele

### Beispiel I-1

### Cyclo(-N-methyl-L-alanyl-D-lactyl-N-methyl-L-isoleucyl-D-4-morpholino-phenyllactyl-N-methyl-L-isoleucyl-D-lactyl-)

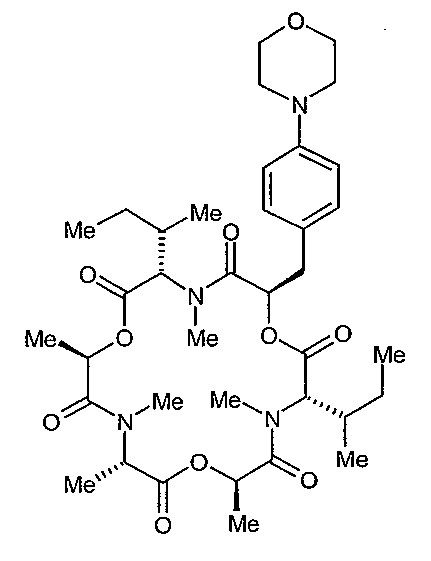

In ein Gemisch aus 103 mg (1,47 mmol) 2,5-Dihydrofuran, 0,8 ml Methanol und 3,1 ml Dichlormethan wird bei -60°C Ozon-Gas eingeblasen, bis die Reaktionsmischung eine bläuliche Farbe aufweist (Olefinverbrauch). Anschließend wird das überschüssige Ozon mit einem Argonstrom (durch Kaliumiodid geleitet) vertrieben. Man gibt 185 mg (2,94 mmol) Natriumcyanoborhydrid zu der Lösung und rührt das Reaktionsgemisch 10 Minuten bei -50°C. Danach wird eine Lösung aus 650 mg (1,00 mmol) Cyclo(-N-methyl-L-alanyl-D-lactyl-N-methyl-L-isoleucyl-D-4-amino-phenyllactyl-N-methyl-L-isoleucyl-D-lactyl-) in 3,9 ml Methanol tropfenweise hinzugegeben und weitere 20 Stunden bei 0°C gerührt. Die Reaktion wird mit 59 mg (0,98 mmol) Essigsäure gequenched. Nach Entfernung des Lösungsmittels im Vakuum werden 7,5 ml gesättigte Natriumbicarbonat-Lösung hinzugegeben. Das Reaktionsgemisch wird 3 mal mit 7,5 ml Dichlormethan extrahiert. Anschließend wird die organische Phase mit gesättigter Natriumchlod-lösung gewaschen und über Natriumsulfat getrocknet. Nach dem einengen im Vakuum wird der zurückbleibende Rückstand an Kieselgel mit dem Laufmittelgemisch Cyclohexan:Aceton (2:1) chromatographiert (Kieselgel 60-Merck, 0,04-0,063 mm). Man erhält 200 mg (27,7% d. Theorie) Cyclo(-N-methyl-L-alanyl-D-lactyl-N-methyl-L-isoleucyl-D-4-morpholino-phenyl-lactyl-N-methyl-L-isoleucyl-D-lactyl-).

HPLC (0,1 % Phosphorsäure / Acetonitril; Gradient: 90/10 (1) 5 %/min, 5/95 (6); Flow: 1,5 ml/min; UV: 210 nm): Rₜ = 12,57 min; Log P-Wert 3,58.

¹H-NMR (CDCl₃, δ): 3,10 (m, 4H, CH₂-N-CH₂-); 3,85 (m, 4H, CH₂-O-CH₂-) ppm.

¹³C-NMR (CDCl₃, δ): 10,5, 10,7, 13,4, 15,5, 15,6, 16,0, 16,9 (7 x CH₃); 29,9, 32,2 (CH₂); 32,6, 34,2 (2 x CH); 30,8, 32,6, 34,2 (3 x NCH₃); 36,4 (CH₂Ph); 49,4 (2 x NCH₂); 55,9, 59,5; 61,1 (3 x NCH); 66,8 (2 x OCH₂); 66,0, 67,5, 70,0 (3 x OCH); 115,7, 130,4 (4 x Ph-C); 126,2 (Ph-C); 150,2 (Ph-C-Mor); 170,2, 170,3, 170,5 (3 x O-C=O); 168,2, 168,6, 169,6 (3 x N-C=O) ppm.

EI-MS m/z (%): 716 (M⁺, 100), 176 (42).

### Beispiel 1-2

### Cyclo(-N-methyl-L-alallyl-D-lactyl-N-methyl-L-isoleucyl-D-4-(2-hydroxyethylsulfonyl-ethylaminophenyl)lactyl-N-methyl-L-isoleucyl-D-lactyl-)

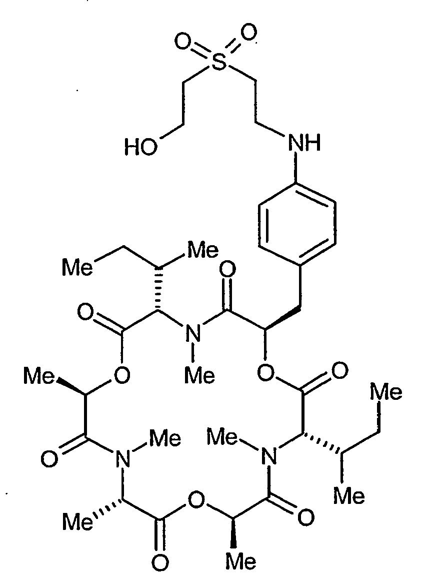

In ein Gemisch aus 80,16 mg (0,67 mMol) 3-Sulfolen, 0,36 ml Methanol und 1,44 ml Dichlormethan wird bei -60°C Ozon-Gas eingeblasen, bis die Reaktionsmischung eine bläuliche Farbe aufweist (Olefinverbrauch). Anschließend wird das überschüssige Ozon mit einem Argonstrom (durch Kaliumiodid geleitet) vertrieben. Man gibt 82,26 mg (1,36 mMol) Natriumcyanoborhydrid zu der Lösung und rührt das Reaktionsgemisch 10 Minuten bei -50°C. Danach wird eine Lösung aus 650 mg (1,00 mmol) Cyclo(-N-methyl-L-alanyl-D-lactyl-N-methyl-L-isoleucyl-D-4-aminophenyllactyl-N-methyl-L-isoleucyl-D-lactyl-) in 1,8 ml absolutem Methanol tropfenweise hinzugegeben und weitere 20 Stunden bei 0°C gerührt. Die Reaktion wird mit 27,13 mg Essigsäure gequenched. Nach Entfernung des Lösungsmittels im Vakuum werden 7,5 ml gesättigte Natriumbicarbonat-Lösung hinzugegeben. Das Reaktionsgemisch wird 3 mal mit 7,5 ml Dichlormethan extrahiert. Anschließend wird die organische Phase mit gesättigter Natriumchlod-lösung gewaschen und über Natriumsulfat getrocknet. Nach dem einengen im Vakuum wird der zurückbleibende Rückstand an Kieselgel mit dem Laufmittelgemisch Cyclohexan:Aceton (1:2) chromatographiert (Kieselgel 60-Merck, 0,04-0,063 mm). Man erhält 90 mg (24,8 % d. Theorie) Cyclo(-N-methyl-L-alanyl-D-lactyl-N-methyl-L-isoleucyl-D-4-(2-hydroxyethyl-sulfonyethylaminophenyl)lactyl-N-methyl-L-isoleucyl-D-lactyl-).

¹H-NMR (CDCl₃, δ): 3,30 (m, 2H, CH₂-OH); 3,46 (m, 2H, NH-CH₂-); 3,69 (m, 2H, SO₂-CH₂-); 4,11 (m, 2H, CH₂-SO₂-) ppm.

¹³C-NMR (CDCl₃, δ): 10,5, 10,5, 13,7, 15,4, 15,5, 16,3, 16,6 (7 x CH₃); 24,7, 25,0 (CH₂); 30,8, 31,7 (2 x CH); 32,7, 32,9, 33,9 (3 x NCH₃); 37,5 (CH₂Ph); 37,5 (HNCH₂); 53,7, 56,3 (2 x SO₂CH₂); 56,3 (CH₂OH); 54,6, 59,8, 60,7 (3 x NCH); 66,5, 67,2, 70,4 (3 x OCH); 113,1, 130,5 (4x Ph-C); 124,1 (Ph-C); 146,0 (Ph-C-NH); 169,2, 170,3, 170,4, (3 x O-C=O); 169,5, 170,4, 170,5 (3 x N-C=O) ppm.

Negativ-ESI-MS *m*/*z (%):* 781 (M⁺-H, 36).

Positiv-ESI-MS *mlz (%):* 781 (M⁺+H, 42).

### Cyclo(-N-methyl-L-alanyl-D-lactyl-N-methyl-L-isoleucyl-D-amino-phenyllacty-N-methyl-L-isoleucyl-D-lactyl-)

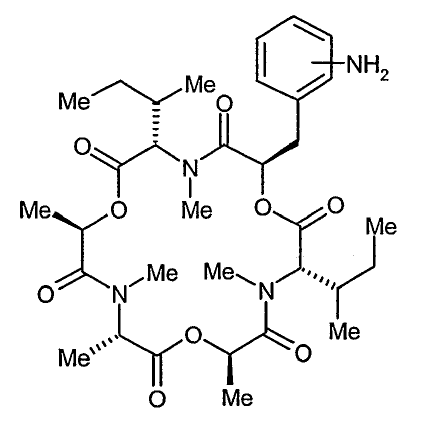

Man verrührt 1,0g (1,48 mmol) Cyclo(-N-methyl-L-alanyl-D-lactyl-N-methyl-L-isoleucyl-D-2-(3- und 4-)-amino-phenyllactyl-N-methyl-L-isoleucyl-D-lactyl-) in 75 ml Ethanol, versetzt mit 0,15 g Hydrierkatalysator (20 % Palladiumhydroxyd-Kohle) und hydriert das Reaktionsgemisch 4 Stunden bei Raumtemperatur unter Normaldruck. Anschließend wird der Katalysator abfiltriert und das Lösungsmittel im Vakuum abdestilliert. Der verbleibende Rückstand enthält ein Isomerengemisch, das mittels Craig-Verteilung aufgetrennt werden kann:
Apparatur: 25 ml, 200 Verteilungselemente (Fa. Labortec)
Verteilungssystem: Essigsäureethylester-n-Heptan-DMF-Wasser (4:6:5:5)
Phasenverhältnis: 1
Trennstufen: n = 250, anschließend 300 (Kreislauf)

Aufarbeitung: Nach Beendigung des 1. Verteilungszyklus (n = 250) wurde der Inhalt jedes 10. Elementes entnommen, die Lösungsmittel am Rotationsverdampfer abrotiert und der Rückstand, nach Wägung, in 0,5-1,0 ml Acetonitril aufgenommen und mittels analytischer HPLC untersucht. Das Cyclo(-N-methyl-L-alanyl-D-lactyl-N-methyl-L-isoleucyl-D-3-amino-phenyllactyl-N-methyl-L-isoleucyl-D-lactyl-) befand sich im Auslauf A1. Der Inhalt wurde entnommen und bei 40°C am Rotationsverdampfer bis zur Trockne eingeengt. Ein Verteilungszyclus von n = 300 (Keislauf) schloss sich an. Nach beendeter Verteilung wurde der Inhalt der Elemente E 90-130 entnommen und bei 40°C im Vakuum eingeengt - es handelte sich um das Cyclo(-N-methyl-L-alanyl-D-lactyl-N-methyl-L-isoleucyl-D-4-amino-phenyllactyl-N-methyl-L-isoleucyl-D-lactyl-). In analoger Weise konnte aus den Elementen E 155-180 das entsprechende Cyclo(-N-methyl-L-alanyl-D-lactyl-N-methyl-L-isoleucyl-D-2-amino-phenyllactyl-N-methyl-L-isoleucyl-D-lactyl-)erhalten werden.

Analytische HPLC:
Gerät: HP 1090 Fa. Hewlett Packard
Säule: Kromasil 100, C 18, 5 µm, 125 x 4 mm, Stahl
Mobile Phase: Wasser/Acetonitril (A/B)
Gradient: A = 90% / B = 10%, 2 min, 5% B / min, A = 5 % / B = 95 % 6 min isokratisch
Fluss: 1,5 ml / min
Detektion: UV, λ = 210 nm
Temperatur: 40°C
Injektionsvolumen: 3,5 µl

### Beispiel IV-1

### Cyclo(-N-methyl-L-alanyl-D-lactyl-N-methyl-L-isoleucyl-D-2-amino-phenyllactyl-N-methyl-L-isoleucyl-D-lactyl-):

HPLC (0,1 % Phosphorsäure / Acetonitril; Gradient: 90/10 (1) 5%/min, 5/95 (6); Flow: 1,5 ml/min; UV: 210 nm): Rₜ = 11,63 min; Log P-Wert 3,18.
¹³C-NMR (CDCl₃, δ): 10,2, 10,5, 13,3, 15,5, 15,5, 15,8, 17,1 (7 x CH₃); 23,9, 24,4 (CH₂); 26,8, 30,1 (2 x CH); 30,9, 31,5, 32,0 (3 x NCH₃); 34,0 (CH₂Ph); 56,8, 57,9, 60,4 (3 x NCH); 65,5, 67,5, 68,9 (3 x OCH); 116,1, 118,5, 119,1, 128,0, 131,3 (5 x Ph-C); 145,5 (Ph-C-NH₂); 168,5, 169,7, 170,3 (3 O-C=O); 168,6, 170,0, 170,8 (3 x N-C=O) ppm.

### Beispiel IV-2

### Cyclo(-N-methyl-L-alanyl-D-lactyl-N-methyl-L-isoleucyl-D-3-amino-phenyllactyl-N-methyl-L-isoleucyl-D-lactyl-):

HPLC (0,1 % Phosphorsäure / Acetonitril; Gradient: 90/10 (1) 5 %/min, 5/95 (6); Flow: 1,5 ml/min; UV: 210 nm): Rₜ = 9,32 min; Log P-Wert 2,35.
¹³C-NMR (CDCl₃, δ): 10,3, 10,5, 13,3, 15,3, 15,5, 15,9, 16,7 (7 x CH₃); 24,0, 24,6 (CH₂); 29,8, 30,7 (2 x CH); 32,0, 32,5, 34,0 (3 x NCH₃); 37,3 (CH₂Ph); 55,6, 59,5, 61,0 (3 x NCH); 66,0, 67,3, 69,9 (3 x OCH); 113,4, 116,1, 119,2, 129,1, 136,1 (5 x Ph-C); 146,6 (Ph-C-NH₂); 168,2, 169,5, 170,2 (3 x O-C=O), 168,6, 170,0, 170,3 (3 x N-C=O) ppm.

### Beispiel IV-3

### Cyclo(-N-methyl-L-alanyl-D-lactyl-N-methyl-L-isoleucyl-D-4-amino-phenyllactyl-N-methyl-L-isoleucyl-D-lactyl-):

HPLC (0,1 % Phosphorsäure / Acetonitril; Gradient: 90/10 (1) 5 %/min, 5/95 (6); Flow: 1,5 ml/min; UV: 210 nm): Rₜ = 8,39 min; Log P-Wert 2,08.
¹³C-NMR (CDCl₃, δ): 10,3, 10,7, 15,4, 15,6, 15,6, 16,0, 16,8 (7 x CH₃); 24,2, 24,7 (CH₂); 30,7, 32,2 (2 x CH); 32,6, 33,7, 34,1 (3 x NCH₃); 36,5 (CH₂Ph); 55,7, 59,5, 61,2 (3 x NCH); 66,1, 67,4, 70,1 (3 x OCH); 115,1, 130,4 (4 x Ph-C); 124.9 (Ph-C); 145,2 (Ph-C-NH₂); 168,4, 169,6, 170,3 (3 x O-C=O), 168,6, 170,2, 170,4 (3 x N-C=O) ppm.

### Beispiel III-1

### Cyclo(-N-methyl-L-alanyl-D-lactyl-N-methyl-L-isoleucyl-D-4-nitro-phenyllactyl-N-methyl-L-isoleucyl-D-lactyl-)

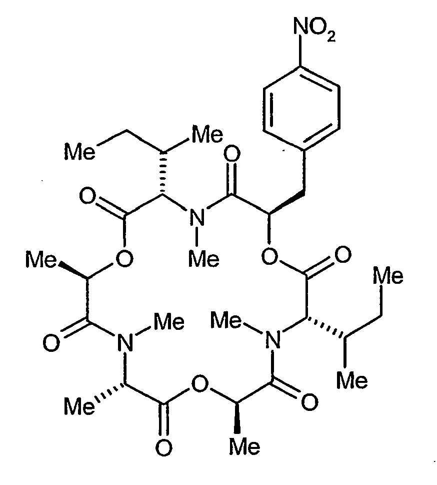

0,5 g (0,79 mmol) Cyclo(-N-methyl-L-alanyl-D-lactyl-N-methyl-L-isoleucyl-D-phenyl-lactyl-N-methyl-L-isoleucyl-D-lactyl-) wurden in einem Kolben auf -10°C gekühlt und innerhalb von 15 Minuten tropfenweise mit 7,0 ml rauchender 98 %iger Salpetersäure versetzt. Nach einer Stunde Rühren bei -10°C wurde das Reaktionsgemisch langsam auf 100 g Eis gegeben und mit gesättigter Natriumbicarbonat-Lösung auf pH 7 eingestellt. Anschließend wurde das Reaktionsgemisch mit Essigsäureethylester extrahiert. Danach wurde die separierte organische Phase mit gesättigter Kochsalzlösung gewaschen und erneut abgetrennt. Nach dem Trocknen über Natriumsulfat wurde das Lösungsmittel im Vakuum abdestilliert. Das zurückbleibende Isomerengemisch wurde mittels präparativer HPLC gereinigt.
Fp.: 122-126°C
¹³C-NMR (CDCl₃, δ): 10,2, 10,5, 15,4, 15,6, 15,6, 15,9, 17,1 (7 x CH₃); 24,2, 24,5
(CH₂); 31,0, 31,5 (2 x CH); 32,2, 34,0, 34,0 (3 x NCH₃); 37,0 (CH₂Ph); 56,4, 59,8, 60,3 (3 x NCH); 65,6, 67,6, 69,4 (3 x OCH); 123,3, 130,3 (4 x Ph-C); 143,3 (Ph-C); 146,9 (Ph-C-NO₂); 167,2, 169,8, 170,2 (3 x O-C=O), 168,2, 169,8, 170,2 (3 x N-C=O) ppm.
EI-MS *m*/*z (%):* 676 (M⁺, 28).

### Röntgenstrukturbestimmung:

Röntgentaugliche Einkristalle konnten durch Umkristallisieren aus einem Lösungsmittelgemisch Chloroform/n-Hexan erhalten werden. Die Bestimmung der Gitterkonstanten und die Erfassung der Reflexintensitäten erfolgte bei -80°C einem Siemens P4-Vierkreisdiffraktometer. Die Strukturauflösung erfolgte mit Hilfe der direkten Methoden (Programmsystem SHELXTL). Die Verfeinerung der Struktur erfolgte mit dem Programm SHELXL-93 gegen F².

### Kristalldaten:

| | |
|---|---|
| C₃₃H₄₈N₄O₁₀ (660,71 g/mol) | Mo K_{α} Strahlung |
| Monoklin | λ = 0,71073 A |
| P2₁ | µ = 0,081 mm⁻¹ |
| a = 9,714 (2) A | T = 193 K |
| b = 15,244 (3) A | 0,4 x 0,2 x 0,2 mm |
| c = 14,279 (2) A | Prisma farblos |
| β = 109,68 (2)° | |
| V = 6237,4 (20) A³ | |
| Z=2 | |
| Dₓ = 1,102 Mg/m³ | |

### Beispiel II-1 (Referenzverbindung, Vorstufe)

### Cyclo(-N-methyl-L-alanyl-D-lactyl-N-methyl-L-isoleucyl-D-phenyllactyl-N-methyl-L-isoleucyl-D-lactyl-)

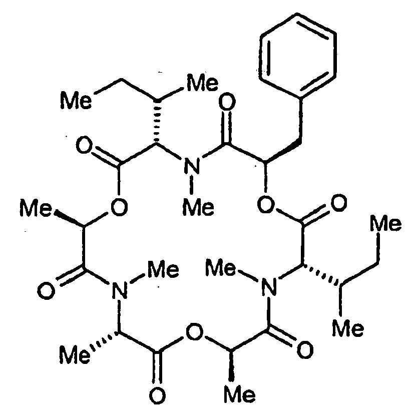

Zu einem Gemisch aus 1,50 g (2,31 mmol) N-Methyl-L-alanyl-D-lactyl-N-methyl-L-isoleucyl-D-phenyllactyl-N-methyl-L-isoleucyl-D-milchsäure (dargestellt in Analogie zu DE 4317458, EP 658551 A1; Jeschke et al. Bioorg. Chem. 1999, S. 207-214), 0,83 g (6,43 mmol) N,N-Diisopropylethylamin (DIEA) in 500 ml Dichlormethan werden bei 0°C unter Rühren 0,70 g (2,78 mmol) Bis(2-oxo-3-oxazolidinyl)phosphoniumsäurechlorid (BOP-Cl) gegeben. Nach 24 Stunden Rühren bei Raumtemperatur werden nochmals 0,83 g (6,43 mmol) DIEA und 0,70 g (2,78 mmol) BOP-Cl hinzugegeben und weitere 24 Stunden gerührt. Anschließend wird das Reaktionsgemisch zweimal mit Wasser gewaschen, die organische Phase abgetrennt und über Natriumsulfat getrocknet. Danach wird die organische Phase im Vakuum eingeengt und das zurückbleibende Rohprodukt mittels Säulenchromatographie (Kieselgel 60-Merck, Korngröße: 0,04-0,063 mm) mit dem Laufmittelgemisch Toluol-Essigsäureethylester (2:1) gereinigt. Man erhält 2,2 g (64,7 % d. Theorie) Cyclo(-N-methyl-L-alanyl-D-lactyl-N-methyl-L-isoleucyl-D-phenyllactyl-N-methyl-L-isoleucyl-D-lactyl-).
HPLC (0,1 % Phosphorsäure / Acetonitril; Gradient: 90/10 (1) 5 %/min, 5/95 (6); Flow: 1,5 ml/min; UV: 210 nm): Rₜ = 13,94 min; Log P-Wert 4,23.
¹³C-NMR (CDCl₃, δ): 10,3, 10,7, 13,4, 15,5, 15,6, 16,0, 16,9 (7 x CH₃); 24,1, 24,7 (CH₂); 29,9, 30,7 (2 x CH); 32,5, 33,9, 34,2 (3 x NCH₃); 37,3 (CH₂Ph); 55,9, 59,5, 61,1 (3 x NCH); 66,0, 67,5, 70,0 (3 x OCH); 126,8 (Ph-C); 128,4, 129,6 (4 x Ph-C); 135,4 (Ph-C); 168,0, 169,6, 170,3 (3 x O-C=O), 168,6, 170,2, 170,5 (3 x N-C=O) ppm.
EI-MS *m*/*z (%):* 631 (M⁺, 52), 558 (22), 415 (26), 330 (10), 258 (89), 100 (90).

### Biologische Beispiele

### Beispiel A

### In vivo Nematodentest

### Haemonchus contortus / Schaf

Experimentell mit *Haemonchus contortus* infizierte Schafe wurden nach Ablauf der Präpatenzzeit des Parasiten behandelt. Die Wirkstoffe wurden als reiner Wirkstoff oral und/oder intravenös appliziert.

Der Wirkungsgrad wird dadurch bestimmt, dass man die mit dem Kot ausgeschiedenen Wurmeier vor und nach der Behandlung quantitativ auszählt.

Ein völliges Sistieren der Eiausscheidung nach der Behandlung bedeutet, dass die Würmer abgetrieben wurden oder so geschädigt sind, dass sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff / Beispiel-Nr. | Dosis effectiva in [mg / kg] |
|---|---|
| I-1 | 0,10 |
| I-2 | 0,10 |
| IV-1 | 0,05 |
| IV-2 | 0,05 |

### Beispiel B

### In vivo Nematodentest

### Trichostrongylus colubriformis / Schaf

Experimentell mit *Trichostrongylus colubriformis* infizierte Schafe wurden nach Ablauf der Präpatenzzeit des Parasiten behandelt. Die Wirkstoffe wurden als reiner Wirkstoff oral und/oder intravenös appliziert.

Der Wirkungsgrad wird dadurch bestimmt, dass man die mit dem Kot ausgeschiedenen Wurmeier vor und nach der Behandlung quantitativ auszählt.

Ein völliges Sistieren der Eiausscheidung nach der Behandlung bedeutet, dass die Würmer abgetrieben wurden oder so geschädigt sind, dass sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff / Beispiel-Nr. | Dosis effectiva in [mg / kg] |
|---|---|
| I-1 | 0,25 |
| IV-2 | 0,25 |

## Patentansprüche

1. Cyclische Depsipeptide der allgemeinen Formel (I) und deren Salze, 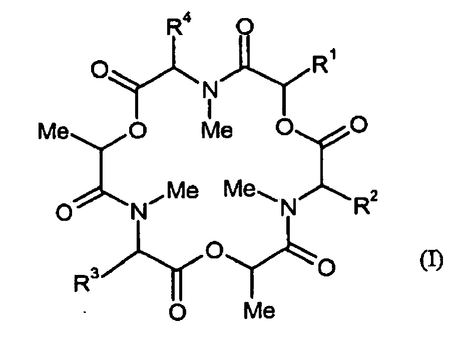 in welcher
R¹ für Nitrobenzyl oder R'R" N-Benzyl steht,
worin
R' und R" unabhängig voneinander jeweils Wasserstoff, gegebenenfalls substituiertes C₁-C₄-Alkyl, Formyl, C₁-C₄-Alkoxy-C₁-C₄-alkyl, C₁-C₄-Alkoxycarbonyl, Hydroxy-C₁-C₂-alkyl-sulfonyl-C₁-C₂-alkyl bedeuten
oder
R' und R" gemeinsam mit dem Stickstoffatom, an das sie gebunden sind, einen gegebenenfalls substituierten mono- oder polycyclischen, gegebenenfalls überbrückten und/oder spirocyclischen, gesättigten oder ungesättigten Heterocyclus bilden, der ein bis drei weitere Heteroatome aus der Reihe Stickstoff, Sauerstoff und Schwefel enthält, oder 'R' und R" gemeinsam C₃-C₅-Alkylenmonocarbonyl oder einen gegebenenfalls substituierten Diacylrest einer C₄-C₆-Dicarbonsäure bilden, und
R², R³ und R⁴ unabhängig voneinander für C₁-C₄-Alkyl stehen,
sowie deren optische Isomere und Racemate.

2. Depsipeptide der allgemeinen Formel (I) und deren Salze gemäß Anspruch 1,
in welcher
R¹ für Nitrobenzyl oder R'R"N-Benzyl stehen,
worin
R' und R" unabhängig voneinander jeweils für Wasserstoff, C₁-C₃-Alkyl, insbesondere Methyl, Ethyl, C₁-C₃-Alkoxy-C₁-C₃-Alkyl, insbesondere Methoxyethyl, 2-Hydroxyethylsulfonyl-C₁-C₂-alkyl insbesondere 2-Hydroxyethyl-sulfonyl-ethyl oder
R' und R" gemeinsam mit dem Stickstoffatom, an das sie gebunden sind, N-Pyrrolidino, N-Piperidino, N-Piperazino, N-Morpholino, N-2,6-Dimethylmorpholino, N-Thiomorpholino, N-Pyrazolo, N-Imidazolo, 2-Oxo-pyrrolidin-1-yl, 2-Oxo-piperidin-1-yl, 2-Oxo-azepan-1-yl-methyl, Succinimino, Maleinimino oder Glutarimino bedeuten,
R², R³ und R⁴ unabhängig voneinander für C₁-C₄-Alkyl stehen,
sowie deren optische Isomere und Racemate.

3. Depsipeptide der allgemeinen Formel (I) und deren Salze gemäß Anspruch 1,
in welcher
R¹ für 4-Nitrobenzyl, 4-Aminobenzyl, 4-Morpholinobenzyl, 4-Hydroxyethyl-sulfonylethylaminobenzyl
R² und R⁴ unabhängig voneinander für C₁-C₄-Alkyl, insbesondere Methyl, Isopropyl, Isobutyl oder sek-Butyl stehen,
R³ für Methyl oder Ethyl steht,
sowie deren optische Isomere und Racemate.

4. Verfahren zur Herstellung der cyclischen Depsipeptide der allgemeinen Formel (I) und deren Salze 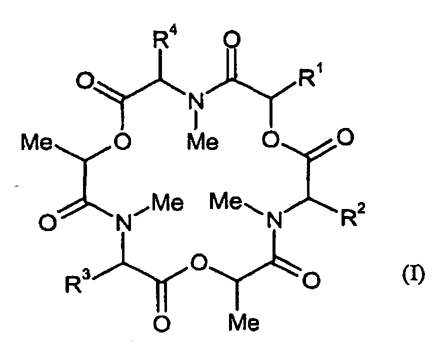 in welcher
R¹, R², R³ und R⁴ die unter Punkt 1 angegebene Bedeutung besitzen,
werden hergestellt, indem man
a) in einem ersten Schritt die cyclischen Depsipeptide der allgemeinen Formel (II) und deren Salze, 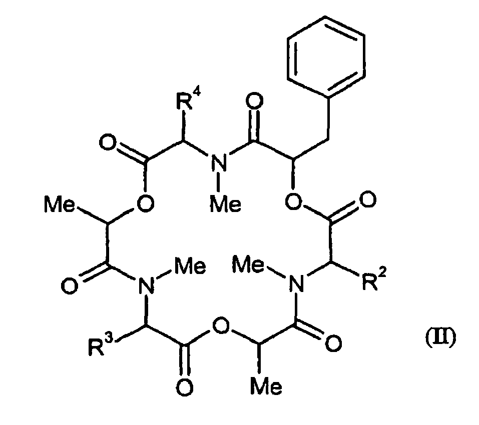 in welcher
R², R³ und R⁴ die unter Punkt 1 angegebene Bedeutung besitzen,
in Gegenwart eines Nitrierungsmittels und gegebenenfalls in Gegenwart eines Verdünnungsmittels nitriert, und
b) gegebenenfalls in einem zweiten Schritt in den so erhaltenen cyclischen Depsipeptiden der allgemeinen Formel (III) oder deren Salzen, 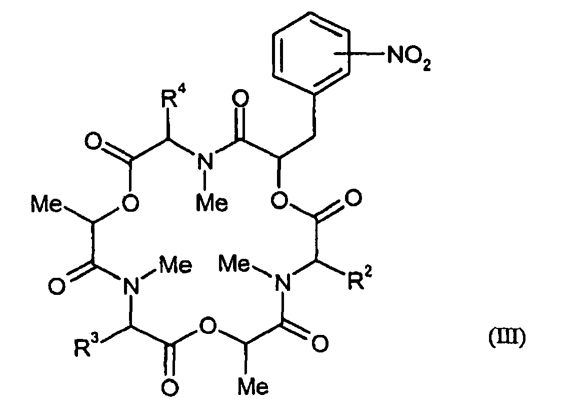 in welcher
R², R³ und R⁴ die unter Punkt 1 angegebene Bedeutung besitzen,
in Gegenwart eines Reduktionsmittels und gegebenenfalls in Gegenwart eines Verdünnungsmittels die Nitrogruppe reduziert, und
c) gegebenenfalls in einem dritten Schritt die cyclischen Depsipeptide der allgemeinen Formel (IV) und deren Salze, 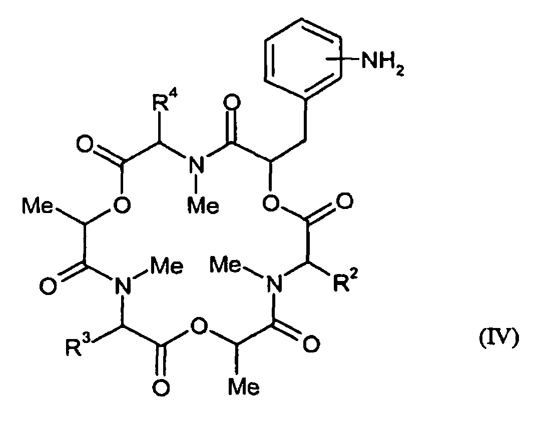 in welcher
R², R³ und R⁴ die unter Punkt 1 angegebene Bedeutung besitzen,
zur Einführung der Reste R' und R" in Gegenwart eines geeigneten Aldehyds und eines Reduktionsmittels und gegebenenfalls in Gegenwart eines Verdünnungsmittels aminoalkyliert, oder
in Gegenwart eines geeigneten Alkylierungsmittels und eines basischen Reaktionshilfsmittels und gegebenenfalls in Gegenwart eines Verdünnungsmittels N-alkyliert, oder
in Gegenwart eines geeigneten Acylierungsmittels und eines basischen Reaktionshilfsmittels und gegebenenfalls in Gegenwart eines Verdünnungsmittels N-acyliert.

5. Mittel enthaltend ein cyclisches Depsipeptid der Formel (I) gemäß Anspruch 1.

6. Verwendung von cyclischen Depsipeptiden der Formel (I) gemäß Anspruch 1 zur Herstellung von Arzneimitteln.

7. Vewendung von cyclischen Depsipeptiden der Formel (I) gemäß Anspruch 6 zur Herstellung von Arzneimitteln zur Bekämpfung von Endoparasiten.

## Claims

1. Cyclic depsipeptides of the general formula (I) and salts thereof 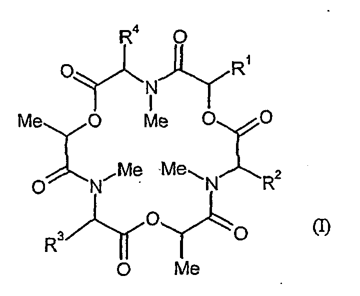 in which
R¹ represents nitrobenzyl or R'R"N-benzyl
where
R' and R" independently of one another each represent hydrogen, optionally substituted C₁-C₄-alkyl, formyl, C₁-C₄-alkoxy-C₁-C₄-alkyl, C₁-C₄-alkoxycarbonyl, hydroxy-C₁-C₂-alkylsulphonyl-C₁-C₂-alkyl
or
R' and R" together with the nitrogen atom to which they are attached form an optionally substituted mono- or polycyclic saturated or unsaturated heterocycle which is optionally bridged and/or spirocyclic and which contains 1 to 3 further heteroatoms from the group consisting of nitrogen, oxygen and sulphur, or R' and R" together form C₃-C₅-alkylenemonocarbonyl or an optionally substituted diacyl radical of a C₄-C₆-dicarboxylic acid, and
R², R³ and R⁴ independently of one another represent C₁-C₄-alkyl,
and optical isomers and racemates thereof.

2. Depsipeptides of the general formula (I) and salts thereof according to Claim 1
in which
R¹ represents nitrobenzyl or R'R"N-benzyl
where
R' and R" independently of one another each represent hydrogen, C₁-C₃-alkyl, in particular methyl, ethyl, C₁-C₃-alkoxy-C₁-C₃-alkyl, in particular methoxyethyl, 2-hydroxyethylsulphonyl-C₁-C₂-alkyl, in particular 2-hydroxyethylsulphonylethyl or
R' and R" together with the nitrogen atom to which they are attached represent N-pyrrolidino, N-piperidino, N-piperazino, N-morpholino, N-2,6-dimethylmorpholino, N-thiomorpholino, N-pyrazolo, N-imidazolo, 2-oxopyrrolidin-1-yl, 2-oxopiperidin-1-yl, 2-oxoazepan-1-ylmethyl, succinimino, maleinimino or glutarimino,
R², R³ and R⁴ independently of one another represent C₁-C₄-alkyl,
and optical isomers and racemates thereof.

3. Depsipeptides of the general formula (I) and salts thereof according to Claim 1
in which
R¹ represents 4-nitrobenzyl, 4-aminobenzyl, 4-morpholinobenzyl,
4-hydroxyethylsulphonylethylaminobenzyl,
R² and R⁴ independently of one another represent C₁-C₄-alkyl, in particular methyl, isopropyl, isobutyl or sec-butyl,
R³ represents methyl or ethyl,
and optical isomers and racemates thereof.

4. Process for preparing the cyclic depsipeptides of the general formula (I) and salts thereof 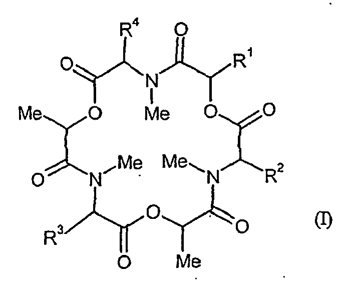 in which
R¹, R², R³ and R⁴ are as defined under item 1
which comprises
a) in a first step, nitrating the cyclic depsipeptides of the general formula (II) and salts thereof 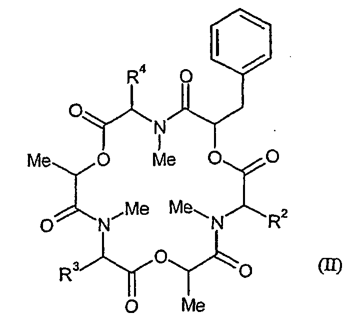 in which
R², R³ and R⁴ are as defined under item 1
in the presence of a nitrating agent and, if appropriate, in the presence of a diluent, and
b) if appropriate, in a second step, reducing the nitro group in the cyclic depsipeptides of the general formula (III) or salts thereof obtained in this manner 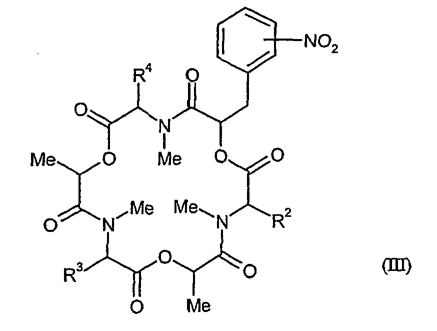 in which
R², R³ and R⁴ are as defined under item 1
in the presence of a reducing agent and, if appropriate, in the presence of a diluent, and
c) if appropriate, in a third step, aminoalkylating the cyclic depsipeptides of the general formula (IV) and salts thereof 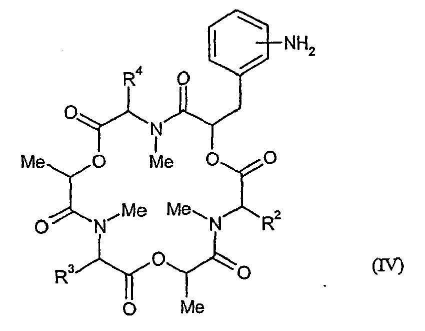 in which
R², R³ and R⁴ are as defined under item 1
to introduce the radicals R' and R", in the presence of a suitable aldehyde and a reducing agent and, if appropriate, in the presence of a diluent, or
N-alkylating these depsipeptides in the presence of a suitable alkylating agent and a basic reaction auxiliary and, if appropriate, in the presence of a diluent, or
N-acylating these depsipeptides in the presence of a suitable acylating agent and a basic reaction auxiliary and, if appropriate, in the presence of a diluent.

5. Compositions comprising a cyclic depsipeptide of the formula (I) according to Claim 1.

6. Use of cyclic depsipeptides of the formula (I) according to Claim 1 for preparing medicaments.

7. Use of cyclic depsipeptides of the formula (I) according to Claim 6 for preparing medicaments for controlling endoparasites.

## Revendications

1. Depsipeptides cycliques de la formule générale (I) et sels de ceux-ci, 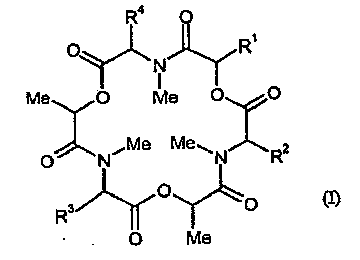 dans lesquels
R¹ désigne un nitrobenzyle ou un groupe R'R"N-benzyle,
dans lequel
R' et R" représentent, indépendamment l'un de l'autre, l'hydrogène, un alkyle en C₁-C₄ le cas échéant substitué, un formyle, un alcoxy en C₁-C₄-alkyle en C₁-C₄, un alcoxycarbonyle en C₁-C₄, un hydroxy-alkylsulfonyle en C₁-C₂-alkyle en C₁-C₂
ou
R' et R" forment conjointement avec l'atome d'azote auquel ils sont liés un hétérocycle mono- ou polycyclique, saturé ou insaturé, le cas échéant substitué, qui est le cas échéant ponté et/ou spirocylique, qui comporte de un à trois autres hétéroatomes de la série de l'azote, de l'oxygène et du soufre, ou R' et R" forment conjointement un alkylènemonocarbonyle en C₃-C₅ ou un radical diacyle le cas échéant substitué d'un acide dicarboxylique en C₄-C₆ et
R², R³ et R⁴ représentent indépendamment les uns des autres un alkyle en C₁-C₄,
ainsi que les racémates et isomères optiques de ceux-ci.

2. Depsipeptides de la formule générale (I) et sels de ceux-ci selon la revendication 1,
dans lesquels
R¹ désigne un nitrobenzyle ou un groupe R'R"N-benzyle,
dans lequel
R' et R" représentent, indépendamment l'un de l'autre, l'hydrogène, un alkyle en C₁-C₃, en particulier un méthyle, un éthyle, un alcoxy en C₁-C₃-alkyle en C₁-C₃, en particulier un méthoxyéthyle, un 2-hydroxyéthylsulfonyl-alkyle en C₁-C₂, en particulier un 2-hydroxyéthyl-sulfonyl-éthyle
ou
R' et R" forment conjointement avec l'atome d'azote auquel ils sont liés un groupe N-pyrrolidino, N-pipéridino, N-pipérazino, N-morpholino, N-2,6-diméthylmorpholino, N-thiomorpholino, N-pyrazolo, N-imidazolo, 2-oxopyrrolidin-1-yle, 2-oxopipéridin-1-yle, 2-oxoazépan-1-yl-méthyle, succinimino, maléinimino ou glutarimino,
R², R³ et R⁴ représentent indépendamment les uns des autres un alkyle en C₁-C₄,
ainsi que les racémates et isomères optiques de ceux-ci

3. Depsipeptides de la formule générale (I) et sels de ceux-ci selon la revendication 1;
dans lesquels
R¹ désigne un groupe 4-nitrobenzyle, 4-aminobenzyle, 4-morpholinobenzyle ou 4-hydroxyéthylsulphonyléthylaminobenzyle,
R² et R⁴ représentent indépendamment l'un de l'autre un alkyle en C₁-C₄, en particulier un méthyle, un isopropyle, un isobutyle ou un sec-butyle,
R³ est un méthyle ou un éthyle,
ainsi que les racémates et isomères optiques de ceux-ci.

4. Procédé de préparation des depsipeptides cycliques de la formule générale (I) et des sels de ceux-ci, 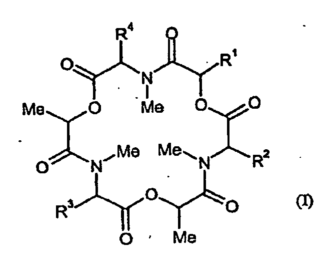 dans lesquels
R¹, R², R³ et R⁴ possèdent la signification indiquée au point 1,
(sont préparés,)
a) dans une première étape, en nitrifiant les depsipeptides cycliques de la formule générale (II) et les sels de ceux-ci 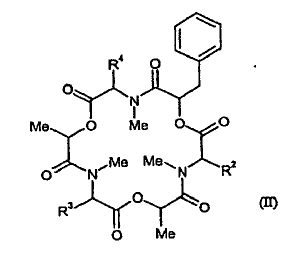 dans lesquels
R², R³ et R⁴ possèdent la signification indiquée au point 1,
en présence d'un agent nitrant et, le cas échéant, en présence d'un diluant, et
b) le cas échéant, dans une deuxième étape, en réduisant le groupe nitro dans les depsipeptides cycliques de la formule (III) ou dans les sels de ceux-ci ainsi obtenus, 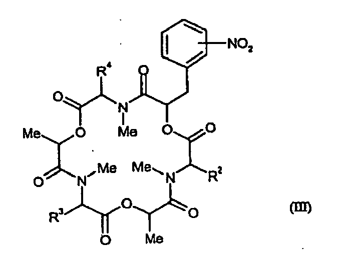 dans lesquels
R², R³ et R⁴ possèdent la signification indiquée au point 1,
en présence d'un agent réducteur et, le cas échéant, en présence d'un diluant, et
c) le cas échéant, dans une troisième étape, en amino-alkylant les depsipeptides de la formule générale (IV) et les sels de ceux-ci 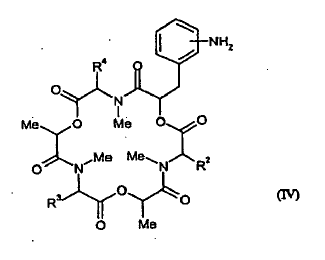 dans lesquels
R², R³ et R⁴ possèdent la signification indiquée au point 1,
afin d'introduire les radicaux R' et R" en présence d'un aldéhyde approprié et d'un agent réducteur et, le cas échéant, en présence d'un diluant, ou
en N-alkylant ces depsipeptides en présence d'un agent d'alkylation approprié et d'un adjuvant de réaction basique et, le cas échéant, en présence d'un diluant, ou
en N-acylant ces depsipeptides en présence d'un agent d'acylation approprié et d'un adjuvant de réaction basique et, le cas échéant, en présence d'un diluant.

5. Agent contenant un depsipeptide cyclique de la formule (I) selon la revendication 1.

6. Utilisation des depsipeptides cycliques de la formule (I) selon la revendication 1 pour la préparation de médicaments.

7. Utilisation des depsipeptides cycliques de la formule (I) selon la revendication 6 pour la préparation de médicaments destinés à combattre les endoparasites.
